# EUROPEAN PATENT APPLICATION

(11) **EP 4 083 212 A1**
(43) Date of publication of application: **02.11.2022**
(21) Application number: 20908021.7
(22) Date of filing: 23.12.2020
(51) Int. Cl.: C12N 15/40, A61K 38/00, A61P 31/14, C07K 14/08, C12N 1/15, C12N 1/19, C12N 1/21, C12N 5/10

(54) **MUTANT RSV F PROTEIN AND USE THEREOF**

(30) Priority: 23.12.2019 US 201962952673 P
(71) Applicant: Mitsubishi Tanabe Pharma Corporation, Osaka-shi, Osaka 541-8505 (JP)
(72) Inventor: HOGIRI, Tomoharu, Osaka-shi, Osaka 541-8505 (JP); KISHIDA, Hiroyuki, Osaka-shi, Osaka 541-8505 (JP); TAKEDOMI, Kei, Osaka-shi, Osaka 541-8505 (JP); BRANDUARDI, Davide, New York, New York 10036 (US); OLOO, Eliud, New York, New York 10036 (US); FEYFANT, Eric, New York, New York 10036 (US); ICHIHARA, Osamu, Tokyo 100-0005 (JP)
(74) Representative: Hoffmann Eitle
(86) International application number: PCT/JP2020/048254
(87) International publication number: WO 2021/132379

(57) **Abstract**

The present invention provides a mutant RSV F protein having a mutation, wherein the mutation is a substitution of a leucine corresponding to a leucine at position 141 of an amino acid sequence of SEQ ID NO: 1 or a leucine corresponding to a leucine at position 142 with a cysteine, and a substitution of a leucine corresponding to a leucine at position 373 with a cysteine, and a disulfide bond is formed between the cysteines. Further provided is a mutant RSV F protein having a mutation, wherein the mutation is a substitution of a glutamic acid corresponding to a glutamic acid at position 60 of the amino acid sequence of SEQ ID NO: 1 with a non-acidic amino acid.

## Description

### [Technical Field]

The present invention relates to mutant RSV F protein and its use.

### [Technical Background]

Respiratory syncytial virus (RSV) infection is a respiratory infection that is prevalent worldwide. Children under the age of 5 are at high risk of aggravation. RSV infection leads to lower respiratory tract inflammation in more than 30 million people annually, and over 3 million people require hospitalization treatment annually. Therefore, many efforts have been made so far toward the development of a vaccine to prevent RSV infection. In the past, in the 1965 - 66 season, clinical trials (subjects were infants) were conducted using a virus inactivated by formalin as a vaccine antigen. However, instead of reducing infectious diseases, it resulted in a 16-fold increase in hospitalization rates due to RSV infection. One of the causes is thought to be that, during an inactivation treatment, an F antigen, which is one of surface antigens of the virus, was denatured, and only antibodies with low RSV neutralization activity were induced, and thereby, an immune response is hindered. Although various efforts have been made since then, an effective vaccine has not yet been developed.

In such a situation, a study using healthy adult serums was conducted, noting that healthy adults rarely become severely infected with RSV. As a result of the study, it was found that healthy adult serums have a high viral infection neutralization effect, and that the infection neutralization effect is due to an anti-RSV F protein antibody (Non-Patent Document 1). Research on RSV F protein has also been advanced, and it has been found that a mature F protein has a metastable pre-fusion conformation (which may be referred to herein as a "pre-F" or "pre-type F protein") and a most stable post-fusion conformation (which may be referred to herein as a "post-F" or "post-type F protein"), and, due to its instability, the pre-fusion conformation tends to refold early into the post-fusion conformation in a general vaccine formulation solution.

Further, as the structure of the pre-F, which has been difficult to be stably obtained, has become clear (Non-Patent Document 2), it has been found that the pre-type F protein forms a trimer, and that some of epitopes (epitopes Φ, I, II, III, IV, V and the like) present in the pre-type F protein are lost due to a structural change to the post-F.

In a study that examines adult human serum in more detail, it has become clear that, among anti-F protein antibodies contained in the serum, antibodies with a high viral infection neutralization capacity are mainly antibodies that specifically bind to the epitope Φ and the epitope V which are present in the pre-type F protein, and these antibodies specifically recognize the pre-type F protein but do not bind to the post-F, and that the above serum also contains an antibody that also binds to the post-F, but has a low viral infection neutralization capacity (Non-Patent Document 3). Further, an infant with inadequate immune maturation is at a high risk of exacerbation of symptoms during infection. However, this is caused by that an infant who is initially infected or who has a low frequency of infection has little ability to induce immunity targeting the epitope Φ or the epitope V. Efficient induction of an antibody having a high viral infection neutralization capacity in a vaccine antigen is important, and an ideal vaccine antigen is thought to be an antigen that can preferentially induce an antibody against the epitope Φ or the epitope V having a neutralization capacity.

Attempts have been made to stabilize the pre-type F protein having the epitope Φ or the epitope V by a mutation or the like that introduces an artificial disulfide bond into the RSV F protein. For example, a DS-Cavl mutant (Patent Document 1, Non-Patent Document 5, Non-Patent Document 6) or other mutants (Patent Document 2) have been reported. However, the stabilization of the pre-type F protein is not sufficient, and research on this is still ongoing.

### [Related Art]

### [Patent Document]

[Patent Document 1] International Publication No. 2017/172890.
[Patent Document 2] International Publication No. 2017/109629.

### [Non-Patent Documents]

[Non-Patent Document 1] Ngwuta J. O. et al., Science Translational Medicine 2015; 309: 309ra162.
[Non-Patent Document 2] Jason S. McLellan et al., Science 2013; 340: 1113.
[Non-Patent Document 3] Morgan S. A. Gilman et al., 2016, Science Immunology, 1 (6), pii: eaaj1879.
[Non-Patent Document 4] Eileen Goodwin et al., Immunity 2018; 48 (2): 339.
[Non-Patent Document 5] Jason S. McLellan et al., Science 2013; 342:592.
[Non-Patent Document 6] M. Gordon Joyce et al., Nature structural and molecular biology 2016; 23 (9): 811.

### [Summary of the Invention]

### [Problems to Be Solved by the Invention]

The present invention is intended to provide a technique for efficiently inducing an antibody having a high RSV infection neutralization capacity.

### [Means for Solving the Problems]

The present inventors have found that a mutant RSV F protein including a specific amino acid mutation efficiently induces an antibody having a high RSV infection neutralization capacity, and thus have accomplished the present invention.

The present invention is as follows.
[1] A mutant RSV F protein having a mutation, wherein the mutation is a substitution of a leucine corresponding to a leucine at position 141 of an amino acid sequence of SEQ ID NO: 1 or a leucine corresponding to a leucine at position 142 with a cysteine, and a substitution of a leucine corresponding to a leucine at position 373 with a cysteine, and a disulfide bond is formed between the cysteines.
[2] The mutant RSV F protein according to [1], the mutant RSV F protein being derived from an RSV subtype A or an RSV subtype B.
[3] The mutant RSV F protein according to [2], wherein the RSV subtype A is an RSV A2 strain or an RSV Long strain.
[4] The mutant RSV F protein according to [2], wherein the RSV subtype B is an RSV 18537 strain.
[5] The mutant RSV F protein according to any one of [1] - [4] wherein the mutant RSV F protein comprises an amino acid sequence having an identity of 85% or more to SEQ ID NO: 2 wherein a leucine corresponding to a leucine at position 141 of the amino acid sequence of SEQ ID NO: 1 or a leucine corresponding to a leucine at position 142 of the amino acid sequence of SEQ ID NO: 1, and a leucine corresponding to a leucine at position 373 of the amino acid sequence of SEQ ID NO: 1 are substituted with cysteines, and a disulfide bond is formed between the cysteines, thereby having an ability to induce an antibody against an RSV pre-type F protein.
[6] The mutant RSV F protein according to any one of [1] - [4], wherein, further, an amino acid that forms a furin recognition site that exists on a C-terminal side of a pep27 region is substituted such that the furin recognition site is not recognized by furin.
[7] The mutant RSV F protein according to [6], wherein the amino acid that forms the furin recognition site is an amino acid selected from a group consisting of an arginine corresponding to an arginine at position 133, an arginine corresponding to an arginine at position 135, and an arginine corresponding to an arginine at position 136 of the amino acid sequence of SEQ ID NO: 1, and the amino acid is substituted with a non-basic amino acid.
[8] The mutant RSV F protein according to [6] or [7], wherein the mutant RSV F protein comprises an amino acid sequence having an identity of 85% or more to SEQ ID NO: 3 wherein a leucine corresponding to a leucine at position 141 of the amino acid sequence of SEQ ID NO: 1 or a leucine corresponding to a leucine at position 142 of the amino acid sequence of SEQ ID NO: 1, and a leucine corresponding to a leucine at position 373 of the amino acid sequence of SEQ ID NO: 1 are substituted with cysteines, and further, an amino acid selected from a group consisting of an arginine corresponding to an arginine at position 133 of the amino acid sequence of SEQ ID NO: 1, an arginine corresponding to an arginine at position 135 of the amino acid sequence of SEQ ID NO: 1, and an arginine corresponding to an arginine at position 136 of the amino acid sequence of SEQ ID NO: 1 is substituted with a non-basic amino acid, and a disulfide bond is formed between the cysteines, thereby having an ability to induce an antibody against an RSV pre-type F protein.
[9] The mutant RSV F protein according to [7] or [8], wherein the non-basic amino acid is an asparagine.
[10] The mutant RSV F protein according to any one of [1] - [9], wherein, further, a threonine corresponding to a threonine at position 189 of the amino acid sequence of SEQ ID NO: 1 and/or a serine corresponding to a serine at position 190 are substituted with hydrophobic amino acids.
[11] The mutant RSV F protein according to [10], wherein the hydrophobic amino acids are each independently selected from a group consisting of a valine, an isoleucine, and a leucine.
[12] The mutant RSV F protein according to any one of [1] - [11], wherein, further, a lysine corresponding to a lysine at position 42 of the amino acid sequence of SEQ ID NO: 1 is substituted with an arginine and/or a valine corresponding to a valine at position 384 is substituted with a threonine.
[13] A mutant RSV F protein having a mutation, wherein the mutation is a substitution of a glutamic acid corresponding to a glutamic acid at position 60 of an amino acid sequence of SEQ ID NO: 1 with a non-acidic amino acid.
[14] The mutant RSV F protein according to [13], wherein the non-acidic amino acid is selected from a group consisting of a methionine, a phenylalanine, a leucine, a threonine, and a serine.
[15] A fusion protein comprising: the mutant RSV F protein according to any one of [1] - [14]; and a multimerization domain fused to a C-terminal of the mutant RSV F protein.
[16] The fusion protein according to [15], wherein the multimerization domain is a foldon domain.
[17] The fusion protein according to [16] comprising an amino acid sequence of any one of SEQ ID NOs: 10 - 13.
[18] A multimer of the mutant RSV F protein according to any one of [1] - [14], wherein the fusion protein according to any one of [15] - [17] is associated via the multimerization domain.
[19] The multimer according to [18] being a trimer.
[20] A particulate product of the mutant RSV F protein according to any one of [1] - [14] or the multimer according to any one of claims [15] - [17], wherein the mutant RSV F protein or the fusion protein contains a particle-forming domain, two or more mutant RSV F proteins or multimers are aggregated via the particle-forming domain to form a particle, and the particle-forming domain is located at a position closer to an epitope I than to an epitope Φ on a steric structure of the mutant RSV F protein or the multimer.
[21] The particulate product according to [20], wherein two or more of the mutant RSV F protein or a fusion proteins for producing a particulate product, in which a particle-forming domain is bound to a C-terminal of the mutant RSV F protein or the fusion protein, are aggregated via the particle-forming domain.
[22] The particulate product according to [20] or [21], wherein the multimer is a trimer consisting of the fusion protein according to [16].
[23] The particulate product according to [22], wherein the particle-forming domain is an Fc domain consisting of an amino acid sequence of SEQ ID NO: 30, and the particle-forming domain aggregates by binding to a Z domain of a protein A immobilized on a VLP derived from a modified HBs antigen.
[24] The particulate product according to [22], wherein the particle-forming domain is a peptide consisting of an amino acid sequence of SEQ ID NO: 42.
[25] A fusion protein for producing a particulate product comprising: the mutant RSV F protein according to any one of [1] - [14], or the fusion protein according to any one of [15] - [17], and a particle-forming domain fused to a C-terminal thereof.
[26] The fusion protein for producing a particulate product according to [25], wherein the particle-forming domain is an Fc domain consisting of an amino acid sequence of SEQ ID NO: 30.
[27] The fusion protein for producing a particulate product according to [25], wherein the particle-forming domain is a peptide consisting of an amino acid sequence of SEQ ID NO: 42.
[28] A polynucleotide encoding the mutant RSV F protein according to any one of [1] - [14], the fusion protein according to any one of [15] - [17], or the fusion protein for producing a particulate product according to any one of [25] - [27].
[29] An expression unit comprising the polynucleotide according to [28].
[30] A host cell comprising the expression unit according to [29].
[31] An immunogen comprising: the mutant RSV F protein according to any one of [1] - [14], the fusion protein according to any one of [15] - [17], the multimer according to any one of [18] - [19], or the particulate product according to any one of [20] - [24].
[32] A pharmaceutical composition comprising the expression unit according to [29] or the immunogen according to [31].
[33] An RSV vaccine comprising the expression unit according to [29] or the immunogen according to [31].
[34] The pharmaceutical composition according to [32] or the RSV vaccine according to [33] being for humans.

### [Effect of the Invention]

According to the present invention, a technique for efficiently inducing an antibody having a high RSV infection neutralization capacity can be provided. As a pre-type F protein, a mutant RSV F protein having a significantly improved stability as compared to a conventional one can be provided. The mutant RSV F protein of the present invention has an effect of inducing a group of antibodies that are thought to have a high effect on virus neutralization in preference to a group of antibodies that are thought to contribute less to virus neutralization, particularly in humans (hereinafter, this effect is referred to as an effect of preferentially inducing a group of antibodies that are thought to have a high effect on virus neutralization).

### [Brief Description of the Drawings]

[Fig. 1] A graph showing storage stability of epitope Φ in Example 1 according to one embodiment of the present invention.
[Fig. 2] A graph showing storage stability of epitope Φ in Example 2 according to one embodiment of the present invention.
[Fig. 3] A graph showing trimerization in Example 2 according to one embodiment of the present invention.
[Fig. 4] A graph showing storage stability of epitope Φ in Example 3 according to one embodiment of the present invention.
[Fig. 5] A graph showing trimerization in Example 3 according to one embodiment of the present invention.
[Fig. 6] A graph showing an expression level in Example 3 according to one embodiment of the present invention.
[Fig. 7] A graph showing storage stability of epitope Φ in Example 4 according to one embodiment of the present invention.
[Fig. 8] A graph showing trimerization in Example 4 according to one embodiment of the present invention.
[Fig. 9] A graph showing storage stability of epitope Φ in Example 5 according to one embodiment of the present invention.
[Fig. 10] A graph showing trimerization in Example 5 according to one embodiment of the present invention.
[Fig. 11] A graph showing a recognition property of epitope Φ in Example 5 according to one embodiment of the present invention.
[Fig. 12] A graph showing results of adsorption tests of virus neutralizing antibodies in human serums in Example 5 according to one embodiment of the present invention.
[Fig. 13] A graph showing results of adsorption tests of virus neutralizing antibodies in human serums in Example 5 according to one embodiment of the present invention.
[Fig. 14] A graph showing results of verification of antibodies induced by mouse immunity in Example 5 according to one embodiment of the present invention.
[Fig. 15] A graph showing storage stability of epitope Φ in Example 6 according to one embodiment of the present invention.
[Fig. 16] A graph showing trimerization in Example 6 according to one embodiment of the present invention.
[Fig. 17] A graph showing a recognition property of epitope I in Example 6 according to one embodiment of the present invention.
[Fig. 18] A graph showing results of verification of antibodies induced by mouse immunity in Example 6 according to one embodiment of the present invention.
[Fig. 19] A graph showing storage stability of epitope Φ in Example 7 according to one embodiment of the present invention.
[Fig. 20] A graph showing trimerization in Example 7 according to one embodiment of the present invention.
[Fig. 21] A graph showing a recognition property of epitope I in Example 7 according to one embodiment of the present invention.
[Fig. 22] A graph showing storage stability of epitope Φ in Example 7 according to one embodiment of the present invention (examination of an insertion linker).
[Fig. 23] A graph showing trimerization in Example 7 according to one embodiment of the present invention (examination of an insertion linker).
[Fig. 24] A graph showing a recognition property of epitope I in Example 7 according to one embodiment of the present invention (examination of an insertion linker).
[Fig. 25] A graph showing particle diameters in Example 7 according to one embodiment of the present invention.
[Fig. 26] A graph showing results of polyacrylamide gel electrophoresis in Example 8 according to one embodiment of the present invention (part 1).
[Fig. 27] A graph showing results of polyacrylamide gel electrophoresis in Example 8 according to one embodiment of the present invention (part 2).
[Fig. 28] A graph showing storage stability of epitope Φ in Example 8 according to one embodiment of the present invention.
[Fig. 29] A graph showing trimerization in Example 8 according to one embodiment of the present invention.
[Fig. 30] A graph showing a recognition property of epitope I in Example 8 according to one embodiment of the present invention.
[Fig. 31] A graph showing particle diameters in Example 8 according to one embodiment of the present invention.
[Fig. 32] A graph showing results of verification of antibodies induced by mouse immunity in Example 8 according to one embodiment of the present invention.
[Fig. 33] A graph showing results of polyacrylamide gel electrophoresis in Example 9 according to one embodiment of the present invention.
[Fig. 34] A graph showing storage stability of epitope Φ in Example 9 according to one embodiment of the present invention.
[Fig. 35] A graph showing a recognition property of epitope I in Example 9 according to one embodiment of the present invention.

### [Mode for Carrying Out the Invention]

One embodiment of the present invention is a mutant RSV F protein having a mutation, wherein the mutation is a substitution of a leucine corresponding to a leucine at position 141 of an amino acid sequence of SEQ ID NO: 1 or a leucine corresponding to a leucine at position 142 with a cysteine, and a substitution of a leucine corresponding to a leucine at position 373 with a cysteine, and a disulfide bond is formed between the cysteines.

A natural RSV F protein is first expressed as an F0 polypeptide (precursor). The F0 polypeptide is processed, after an endoplasmic reticulum migration signal is cleaved, by an intracellular furin-like protease at two sites to produce an F1 polypeptide, an F2 polypeptide, and a Pep27 polypeptide. Here, the Pep27 polypeptide is excised and thus does not form a part of a mature F protein. The F2 polypeptide is derived from an N-terminal side portion of the F0 polypeptide and is linked to the F1 polypeptide via two disulfide bonds. The F1 polypeptide is derived from a C-terminal side portion of the F0 polypeptide and immobilizes a mature F protein to a membrane via a transmembrane domain. Three protomers of an F2-F1 heterodimer form a mature F protein as a trimer, which is a pre-type F protein that has a function of fusing a virus membrane and a target cell membrane.

The mutant RSV F protein of the present invention includes the F2 polypeptide and the F1 polypeptide from an N terminal side. As the F1 polypeptide, an F1 polypeptide that does not include a transmembrane region and an intracellular region (hereinafter, may be referred to as an F1 polypeptide (an extracellular region)) is preferably used. Further, the mutant RSV F protein of the present invention may include a Pep27 polypeptide on an N-terminal side of the F1 polypeptide by eliminating one of furin-like protease recognition sites by a mutation to be described later. Further, it is also possible to include a signal sequence on an N-terminal side of the F2 polypeptide, a linker between the F1 polypeptide and the F2 polypeptide, and a tag used for purification or the like on a C-terminal side thereof. Further, the mutant RSV F protein of the present invention preferably forms a trimer.

The mutant RSV F protein of the present invention is preferably derived from an RSV subtype A or an RSV subtype B.

Specific examples of the RSV subtype A include an RSV A2 strain, an RSV Long strain, an RSV S2 strain, and an RSV line 19 strain, and among them, the RSV A2 strain or the RSV Long strain is preferable.

Specific examples of the RSV subtype B include an RSV 18537 strain, an RSV B1 strain, and an RSV 9320 strain, and among them, the RSV 18537 strain is preferable.

The amino acid sequence of SEQ ID NO: 1 is an amino acid sequence of an F0 polypeptide of an F protein of the RSV A2 strain. With reference to this, structural elements of the F0 polypeptide are described.

In SEQ ID NO: 1, an amino acid sequence of a signal sequence is an amino acid sequence at positions 1 - 25.

In SEQ ID NO: 1, an amino acid sequence of an F2 polypeptide is an amino acid sequence at positions 26 - 109.

In SEQ ID NO: 1, an amino acid sequence of a pep27 region is an amino acid sequence at positions 110 - 136.

In SEQ ID NO: 1, an amino acid sequence of an F1 polypeptide (an extracellular region) is an amino acid sequence at positions 137 - 513.

In SEQ ID NO: 1, an amino acid sequence of an F1 polypeptide (a transmembrane region and an intracellular region) is an amino acid sequence at positions 514 - 574. The amino acid sequence of the F1 polypeptide (a transmembrane region and an intracellular region) may be a sequence having an identity of 85% or more, 90% or 95% or more with the amino acid sequence at positions 514 - 574 of SEQ ID NO: 1.

An RSV F protein can include an F2 polypeptide and an F1 polypeptide (an extracellular region). An example of an RSV F protein that includes an F2 polypeptide and an F1 polypeptide (an extracellular region) is an amino acid sequence of SEQ ID NO: 2 that includes, in this order, an amino acid sequence at positions of 26 - 109 of SEQ ID NO: 1 and an amino acid sequence at positions 137 - 513.

An RSV F protein can include an F2 polypeptide, and an F1 polypeptide (an extracellular region) to which a pep27 region is linked. An example of an RSV F protein that includes, in this order, an F2 polypeptide, and an F1 polypeptide (an extracellular region) to which a pep27 region is linked is an amino acid sequence of SEQ ID NO: 3 that includes an amino acid sequence at positions 26 - 513 of SEQ ID NO: 1.

These polypeptides can be separate polypeptide chains, and preferably they are linked by disulfide bonds. That is, an RSV F protein can be a complex of such polypeptides.

### (Mutation in which leucine is substituted with cysteine)

A mutant RSV F protein of the present invention includes a mutation of a substitution of a leucine corresponding to a leucine at position 141 of the amino acid sequence of SEQ ID NO: 1 or a leucine corresponding to a leucine at position 142 with a cysteine, and of a substitution of a leucine corresponding to a leucine at position 373 with a cysteine. In the mutant RSV F protein, by having the above amino acid mutation, in addition to a naturally occurring disulfide bond, a new disulfide bond is formed between the introduced cysteines, and thereby achieving an effect of improving storage stability (maintaining a pre type) of the epitope Φ. In order to form a trimer, in particular, a leucine corresponding to a leucine at position 141 of SEQ ID NO: 1 is substituted with a cysteine and a leucine corresponding to a leucine at position 373 is substituted with a cysteine.

In the present specification, a mutation position of a mutant RSV F protein is indicated by an amino acid number of a reference sequence represented by SEQ ID NO: 1.

A leucine corresponding to a leucine at position 141 of the amino acid sequence of SEQ ID NO: 1, a leucine corresponding to a leucine at position 142, and a leucine corresponding to a leucine at position 373 refer to a leucine corresponding to a leucine at position 141, a leucine corresponding to a leucine at position 142 and a leucine corresponding to a leucine at position 373 in the amino acid sequence of SEQ ID NO: 1, regardless of an origin of a mutant RSV F protein. That is, for an amino acid sequence of an RSV F protein into which a mutation is to be introduced, when an alignment with the amino acid sequence of SEQ ID NO: 1 is performed, they refer to leucine residues respectively positioned at a leucine at position 141, a leucine at position 142, and a leucine at position 373 of the amino acid sequence of SEQ ID NO: 1.

For example, in a case of an amino acid sequence of a mutant RSV F protein of which an N-terminal side is one amino acid shorter as compared to the amino acid sequence of SEQ ID NO: 1, a leucine at position 141 in the amino acid sequence of SEQ ID NO: 1 is at position 140, and such a leucine can be referred to as a leucine corresponding to a leucine at position 141 in the amino acid sequence of SEQ ID NO: 1.

For an amino acid sequence of an RSV F protein into which a mutation is to be introduced, when an alignment with the amino acid sequence of SEQ ID NO: 1 is performed, in a case where an amino acid other than a leucine is present at a position of a leucine corresponding a leucine at position 141, a leucine corresponding to a leucine at position 142, and/or a leucine at position 373 of the amino acid sequence of SEQ ID NO: 1, an embodiment in which the amino acid is substituted with a cysteine is also included in the mutant RSV F protein of the present invention. Further, in a case where the amino acid other than a leucine is a cysteine, the substitution with a cysteine is not required, and the amino acid may be a cysteine as it is.

One embodiment of the present invention is a mutant RSV F protein that includes an F2 polypeptide and an F1 polypeptide (an extracellular region) produced from a mutant F0 polypeptide that includes an amino acid sequence in which a leucine at position 141 or a leucine at position 142, and a leucine at position 373 are substituted with cysteines in the amino acid sequence of SEQ ID NO: 1, and in which a disulfide bond is formed between the cysteines. Specifically, an amino acid sequence is included in which, in the amino acid sequence of SEQ ID NO: 2, a leucine at position 89 or a leucine at position 90, and a leucine at position 321 are substituted with cysteines.

One embodiment of the present invention is a mutant RSV F protein having an ability to induce an antibody against an RSV pre-type F protein, comprising an F2 polypeptide and an F1 extracellular region polypeptide which are produced from a mutant F0 polypeptide that has an amino acid sequence in which, in an amino acid sequence having an identity of 85% or more, preferably an identity of 90% or more, and more preferably an identity of 95% or more with the amino acid sequence of SEQ ID NO: 1, a leucine corresponding to a leucine at position 141 or a leucine corresponding to a leucine at position 142, and a leucine corresponding to a leucine at position 373 of the amino acid sequence of SEQ ID NO: 1 are substituted with cysteines, wherein a disulfide bond is formed between the cysteines. Specifically, an amino acid sequence is included in which, in an amino acid sequence having an identity of 85% or more, preferably an identity of 90% or more, and more preferably an identity of 95% or more with the amino acid sequence of SEQ ID NO: 2, a leucine corresponding to a leucine at position 141 (position 89 in SEQ ID NO: 2) or a leucine corresponding to a leucine at position 142 (position 90 in SEQ ID NO: 2), and a leucine corresponding to a leucine at position 373 (position 321 in SEQ ID NO: 2) of the amino acid sequence of SEQ ID NO: 1 are substituted with cysteines.

In the present specification, a "percent (%) identity" regarding an amino acid sequence is defined as a percentage of amino acid residues in a candidate sequence that are identical to amino acid residues of a specific reference polypeptide sequence after aligning the sequences, introducing gaps when necessary in order to obtain a maximum % identity, and not considering any conservative substitution as a part of a sequence identity. Alignment for a purpose of measuring the % identity can be achieved using various methods within skills of a person skilled in the art, for example, publicly available computer software such as BLAST, BLAST-2, ALIGN, or Megalign (DNASTAR) software. A person skilled in the art can determine appropriate parameters for aligning the sequences, including any algorithm required to achieve a maximum alignment with respect to full lengths of the sequences being compared. However, for a purpose here, a % identity value is obtained using a sequence comparison computer program BLAST in a pairwise alignment. In a situation where BLAST is used for an amino acid sequence comparison, a % identity of a given amino acid sequence A with a given amino acid sequence B is calculated as follows: $100 times a ratio X / Y$ where X is the number of amino acid residues with scores consistent with being identical as determined by a program alignment of A and B of the sequence alignment program BLAST, and Y is the total number of amino acid residues of B. When the length of the amino acid sequence A is different from the length of the amino acid sequence B, it is understood that the % identity of A with respect to B is different from the % identity of B with respect to A. Unless otherwise noted, here, all % identity values are obtained using the BLAST computer program as described in the immediately preceding paragraph.

An amino acid sequence having a predetermined identity with a predetermined amino acid sequence is due to a substitution, a deletion, an insertion, or an addition in the predetermined amino acid sequence. The substitution is preferably a conservative substitution. The "conservative substitution" is that an amino acid residue is substituted with another chemically similar amino acid residue such that an activity of a protein is substantially not altered. Examples thereof include a case where a hydrophobic residue is substituted with another hydrophobic residue, a case where a polar residue is substituted with another polar residue having the charge, and the like. As examples of functionally similar amino acids capable of performing such substitutions, examples of non-polar (hydrophobic) amino acids include an alanine, a valine, an isoleucine, a leucine, a proline, a tryptophan, a phenylalanine, a methionine, and the like. Examples of polar (neutral) amino acids include a glycine, a serine, a threonine, a tyrosine, a glutamine, an asparagine, a cysteine, and the like. Examples of positively charged (basic) amino acids include an arginine, a histidine, a lysine, and the like. Further, examples of negatively charged (acidic) amino acids include an aspartic acid, a glutamic acid, and the like.

### (Mutation of a glutamic acid at position 60)

Another embodiment of the mutant RSV F protein of the present invention is a mutant RSV F protein having a mutation, wherein the mutation is a substitution of a glutamic acid corresponding to a glutamic acid at position 60 of the amino acid sequence of SEQ ID NO: 1 with a non-acidic amino acid.

The non-acidic amino acid is not limited as long as the mutant RSV F protein achieves an effect of preferentially inducing a group of antibodies that are thought to have a high effect on improving storage stability of the epitope Φ, promoting trimerization, and virus neutralization. However, specifically, examples thereof include neutral amino acids, basic amino acids, and non-polar (hydrophobic) amino acids. More specifically, examples of the neutral amino acids include a glycine, a serine, a threonine, a tyrosine, a glutamine, an asparagine, and a cysteine; examples of the basic amino acids include an arginine, a histidine, and a lysine; and examples of the non-polar (hydrophobic) amino acids include an alanine, a valine, an isoleucine, a leucine, a proline, a tryptophan, a phenylalanine, and a methionine.

The non-acidic amino acid is preferably a neutral amino acid or a non-polar (hydrophobic) amino acid.

Further, among the neutral amino acids, a threonine and a serine are preferable, and among the non-polar (hydrophobic) amino acids, it is preferable to select from a group consisting of a methionine, a phenylalanine and a leucine. Therefore, the non-acidic amino acid is preferably selected from a group consisting of a methionine, a phenylalanine, a leucine, a threonine, and a serine.

One embodiment of the present invention is a mutant RSV F protein that includes an F2 polypeptide and an F1 polypeptide (an extracellular region) produced from a mutant F0 polypeptide that includes an amino acid sequence in which a glutamic acid at position 60 of the amino acid sequence of SEQ ID NO: 1 is substituted with a non-acidic amino acid. Specifically, an amino acid sequence is included in which, in the amino acid sequence of SEQ ID NO: 2, a glutamic acid at position 35 is substituted with a non-acidic amino acid.

One embodiment of the present invention is a mutant RSV F protein having an ability to induce an antibody against an RSV pre-type F protein, comprising an F2 polypeptide and an F1 polypeptide (an extracellular region) which are produced from a mutant F0 polypeptide that has an amino acid sequence in which, in an amino acid sequence having an identity of 85% or more, preferably an identity of 90% or more, and more preferably an identity of 95% or more with the amino acid sequence of SEQ ID NO: 1, a glutamic acid corresponding to a glutamic acid at position 60 of the amino acid sequence of SEQ ID NO: 1 is substituted with a non-acidic amino acid. Specifically, an amino acid sequence is included in which, in an amino acid sequence having an identity of 85% or more, preferably an identity of 90% or more, and more preferably an identity of 95% or more with the amino acid sequence of SEQ ID NO: 2, a glutamic acid corresponding to a glutamic acid at position 60 (position 35 in SEQ ID NO: 2) of the amino acid sequence of SEQ ID NO: 1 is substituted with a non-acidic amino acid.

The mutation of the glutamic acid at position 60 may be introduced together with the above-described mutations in which leucines are substituted with cysteines.

### (Mutation of furin recognition site)

The mutant RSV F protein of the present invention may be a mutant RSV F protein wherein, further, an amino acid that forms a furin recognition site on a C-terminal side of a pep27 region is substituted such that the furin recognition site is not recognized by furin. By having such an amino acid substitution, the mutant RSV F protein has an F2 polypeptide and an F1 polypeptide (an extracellular region) to which a pep27 region is linked, and thereby achieving an effect that trimerization is promoted while the storage stability of the epitope Φ is maintained.

The pep27 region is a region corresponding to positions 110 - 136 of the amino acid sequence of SEQ ID NO: 1, and a first recognition site on an N-terminal side thereof and a second recognition site on a C-terminal side thereof are cleaved by furin in a cell. The N-terminal side of the pep27 region is the F2 polypeptide, and the C-terminal side is the F1 polypeptide, and, as a result of the cleavage, the F2 polypeptide and the F1 polypeptide are produced, and these polypeptides form a disulfide bond between cysteine residues that are respectively inherent in the polypeptides to form a complex. Therefore, normally, an RSV F protein does not include a pep27 region. However, due to the mutation, the second recognition site is not cleaved, and only the first recognition site that exists between the C-terminal side of the F2 polypeptide and the pep27 region is cleaved by furin. As a result of the cleavage, a polypeptide including the F1 polypeptide to which the pep27 region is linked and the F2 polypeptide are formed, and these polypeptides form a disulfide bond between the cysteine residues that are respectively inherent in the polypeptides to form a complex. Therefore, the present embodiment may be a complex that includes a polypeptide fragment including the F1 polypeptide to which the pep27 region is linked and the F2 polypeptide.

The substitution may be a substitution of one or more amino acids among the amino acids that form the furin recognition sites. Specifically, the amino acids that form the furin recognition sites are one or more amino acids selected from a group consisting of an arginine corresponding to an arginine at position 133, an arginine corresponding to an arginine at position 135, and an arginine corresponding to an arginine at position 136 of the amino acid sequence of SEQ ID NO: 1, and the amino acids are preferably substituted with non-basic amino acids.

The arginine corresponding to the arginine at position 133, the arginine corresponding to the arginine at position 135, and the arginine corresponding to the arginine at position 136 of the amino acid sequence of SEQ ID NO: 1 may each be independently substituted with a non-basic amino acid.

The non-basic amino acid is not limited as long as an effect that trimerization is promoted while the storage stability of the epitope Φ is maintained. However, specifically, examples thereof include neutral amino acids, acidic amino acids, and non-polar (hydrophobic) amino acids. More specifically, examples of the neutral amino acids include a glycine, a serine, a threonine, a tyrosine, a glutamine, an asparagine, and a cysteine; examples of the acidic amino acids include an aspartic acid and a glutamic acid; and examples of the non-polar (hydrophobic) amino acids include an alanine, a valine, an isoleucine, a leucine, a proline, a tryptophan, a phenylalanine, and a methionine. Among them, the neutral amino acids are preferable, and the asparagine is more preferable.

One embodiment of the mutant RSV F protein of the present invention includes an amino acid sequence in which, in the amino acid sequence of SEQ ID NO: 3, a leucine at position 116 or a leucine at position 117, and a leucine at position 348 are substituted with cysteines, and, an amino acid selected from a group consisting of an arginine at position 108, an arginine at position 110, and an arginine at position 111 is substituted with a non-basic amino acid.

One embodiment of the mutant RSV F protein of the present invention includes an amino acid sequence in which, in an amino acid sequence having an identity of 85% or more, preferably an identity of 90% or more, and more preferably an identity of 95% or more with the amino acid sequence of SEQ ID NO: 3, a leucine corresponding to a leucine at position 141 (leucine at position 116 in SEQ ID NO: 3) or a leucine corresponding to a leucine at position 142 (leucine at position 117 in SEQ ID NO: 3), and a leucine corresponding to a leucine at position 373 (leucine at position 348 in SEQ ID NO: 3) of the amino acid sequence of SEQ ID NO: 1 are substituted with cysteines, and an amino acid selected from a group consisting of an arginine corresponding to an arginine at position 133 (arginine at position 108 in SEQ ID NO: 3), an arginine corresponding to an arginine at position 135 (arginine at position 110 in SEQ ID NO: 3), and an arginine corresponding to an arginine at position 136 (arginine at position 111 in SEQ ID NO: 3) of the amino acid sequence of SEQ ID NO: 1 is substituted with a non-basic amino acid.

Another embodiment of the mutant RSV F protein of the present invention includes an amino acid sequence, in which, in the amino acid sequence of SEQ ID NO: 3, a glutamic acid at position 35 is substituted with a non-acidic amino acid, and, an amino acid selected from a group consisting of an arginine at position 108, an arginine at position 110, and an arginine at position 111 is substituted with a non-basic amino acid.

Another embodiment of the mutant RSV F protein of the present invention includes an amino acid sequence in which, in an amino acid sequence having an identity of 85% or more, preferably an identity of 90% or more, and more preferably an identity of 95% or more with the amino acid sequence of SEQ ID NO: 3, a glutamic acid corresponding to a glutamic acid at position 60 in the amino acid sequence of SEQ ID NO: 1 (a glutamic acid at position 35 in SEQ ID NO: 3) is substituted with a non-acidic amino acid, and an amino acid selected from a group consisting of an arginine corresponding to an arginine at position 133 (arginine at position 108 in SEQ ID NO: 3), an arginine corresponding to an arginine at position 135 (arginine at position 110 in SEQ ID NO: 3), and an arginine corresponding to an arginine at position 136 (arginine at position 111 in SEQ ID NO: 3) of the amino acid sequence of SEQ ID NO: 1 is substituted with a non-basic amino acid.

In SEQ ID NO: 3 or in an amino acid sequence having an identity of 85% or more, preferably an identity of 90% or more, and more preferably an identity of 95% or more with SEQ ID NO: 3, the pep27 region may be substituted with an artificial linker. A sequence of the artificial linker is not particularly limited, but preferably has an amino acid residue length of 6 - 27, and a sequence containing Gly and/or Ser is preferable. More specifically, examples thereof include Gly-Ser-Gly-Ser-Gly-Ser (SEQ ID NO: 18), (Gly-Gly-Gly-Gly-Ser)₂ (SEQ ID NO: 19), and (Gly-Gly-Gly-Gly-Ser)₃ (SEQ ID NO: 20).

### (Other mutations)

The mutant RSV F protein of the present invention may be a mutant RSV F protein in which, in addition to the mutation in which a leucine corresponding to a leucine at position 141 or a leucine corresponding to a leucine at position 142 and a leucine corresponding to a leucine at position 373 of the amino acid sequence of SEQ ID NO: 1 are substituted with cysteines (hereinafter referred to as a mutation in which leucines are substituted with cysteines), the mutation of a glutamic acid at position 60, and/or the mutation of a furin recognition site described above, further, an amino acid corresponding to a threonine at position 189 and/or an amino acid corresponding to a serine at position 190 of the amino acid sequence of SEQ ID NO: 1 are substituted with hydrophobic amino acids.

The mutant RSV F protein according to the present embodiment achieves an effect that an expression level in an expression system is significantly improved while also maintaining the effect of being excellent in the storage stability of the epitope Φ and promoting trimerization and the effect of preferentially inducing a group of antibodies that are thought to have a high effect on virus neutralization.

An amino acid corresponding to a threonine at position 189 and an amino acid corresponding to serine at position 190 of the amino acid sequence of SEQ ID NO: 1 may each be independently substituted with a hydrophobic amino acid.

Further, the mutant RSV F protein including the substitution is difficult to be recognized by an antibody that is ineffective or poor in viral infection neutralization, in addition to the effect of significantly improving an expression level in an expression system while maintaining the effect of being excellent in the storage stability of the epitope Φ and promoting trimerization and the effect of preferentially inducing a group of antibodies that are thought to have a high effect on virus neutralization. Conversely, when an antibody that is ineffective or poor in viral infection neutralization is induced, there is an expected risk that an immune cell that does not or hardly contribute to viral infection neutralization is activated, resulting in exacerbating symptoms. However, as can be seen from the examples described later, the mutant RSV F protein according to the present embodiment is difficult to be recognized by an antibody that is ineffective or poor in viral infection neutralization, and thus, the risk is lower for the mutant RSV F protein according to the present embodiment than a conventional vaccine.

As a specific example, the mutant RSV F protein according to the present embodiment is difficult to be recognized by an antibody that recognizes the epitope I. Since the epitope I induces an antibody that has a significantly low viral infection neutralization capacity, there is an expected risk that, when the epitope I is recognized by the antibody, an immune cell that hardly contributes to viral infection neutralization is activated and thereby, symptoms are exacerbated. However, as can be seen from the examples described later, for the mutant RSV F protein according to the present embodiment, which is difficult to be recognized by an antibody that recognizes the epitope I, the risk is a lower than a conventional vaccine.

The hydrophobic amino acid is not limited as long as the effect that the mutant RSV F protein is difficult to be recognized by an antibody that is ineffective or poor in viral infection neutralization is achieved in addition to the effect of significantly improving an expression level in an expression system while maintaining the effect of being excellent in the storage stability of the epitope Φ and promoting trimerization and the effect of preferentially inducing a group of antibodies that are thought to have a high effect on virus neutralization. However, specifically, examples thereof include an alanine, a valine, an isoleucine, a leucine, a proline, q tryptophan, a phenylalanine, and a methionine. Among them, a selection from a group consisting of a valine, an isoleucine, and a leucine is preferable.

Position 189 in SEQ ID NO: 1 is an amino acid at position 137 in SEQ ID NO: 2, and position 190 in SEQ ID NO: 1 is an amino acid at position 138 in SEQ ID NO: 2. In SEQ ID NO: 2 or in an amino acid sequence having an identity of 85% or more, 90% or more or 95% or more with SEQ ID NO: 2, a mutation of one or both of these may be introduced in addition to the mutation in which leucines are substituted with cysteines and/or the mutation of a glutamic acid at position 60 described above.

Further, position 189 in SEQ ID NO: 1 is an amino acid at position 164 in SEQ ID NO: 3, and position 190 in SEQ ID NO: 1 is an amino acid at position 165 in SEQ ID NO: 3. In SEQ ID NO: 3 or in an amino acid sequence having an identity of 85% or more, 90% or more or 95% or more with SEQ ID NO: 3, a mutation of one or both of these may be introduced in addition to the mutation in which leucines are substituted with cysteines, the mutation of a glutamic acid at position 60, and/or the mutation of a furin recognition site described above.

One embodiment of the present invention may be a mutant RSV F protein in which, in addition to the mutation in which leucines are substituted with cysteines, the mutation of a glutamic acid at position 60, and/or the mutation of a furin recognition site described above, further, in the amino acid sequence of SEQ ID NO: 1, an amino acid corresponding to a lysine at position 42 is substituted with an arginine, and/or an amino acid corresponding to a valine at position 384 is substituted with a threonine.

The mutant RSV F protein according to the present embodiment achieves an effect that an expression level in an expression system is significantly improved while also maintaining the effect of being excellent in the storage stability of the epitope Φ and promoting trimerization and the effect of preferentially inducing a group of antibodies that are thought to have a high effect on virus neutralization.

Position 42 in SEQ ID NO: 1 is an amino acid at position 17 in SEQ ID NO: 2, and position 384 in SEQ ID NO: 1 is an amino acid at position 332 in SEQ ID NO: 2. In SEQ ID NO: 2 or in an amino acid sequence having an identity of 90% or more with SEQ ID NO: 2, a mutation of one or both of these may be introduced in addition to the mutation in which leucines are substituted with cysteines and/or the mutation of a glutamic acid at position 60 described above, and further in addition to the mutation at position 189 and/or position 190 described above.

Further, position 42 in SEQ ID NO: 1 is an amino acid at position 17 in SEQ ID NO: 3, and position 384 in SEQ ID NO: 1 is an amino acid at position 359 in SEQ ID NO: 3. In SEQ ID NO: 3 or in an amino acid sequence having an identity of 90% or more with SEQ ID NO: 3, a mutation of one or both of these may be introduced in addition to the mutation in which leucines are substituted with cysteines, the mutation of a glutamic acid at position 60, and/or the mutation of a furin recognition site described above, and further in addition to the mutation at position 189 and/or position 190 described above.

The mutant RSV F protein may further include commonly known mutations as long as the mutations do not interfere with the effects of the mutant RSV F protein. For example, a mutation for improving an expression level in an expression system may be further included. Specifically, one or more substitutions selected from a group consisting of a substitution of an amino acid corresponding to a proline at position 102 of the amino acid sequence of SEQ ID NO: 1 with an alanine, a substitution of an amino acid corresponding to an isoleucine at position 379 with a valine, and a substitution of an amino acid corresponding to a methionine at position 447 with a valine can be exemplified.

Position 102 in SEQ ID NO: 1 is an amino acid at position 77 in SEQ ID NO: 2, position 379 in SEQ ID NO: 1 is an amino acid at position 327 in SEQ ID NO: 2, and position 447 in SEQ ID NO: 1 is an amino acid at position 395 in SEQ ID NO: 2. In SEQ ID NO: 2 or in an amino acid sequence having an identity of 85%, 90%, or 95% or more with SEQ ID NO: 2, a mutation of one or both of these may be introduced in addition to the mutation in which leucines are substituted with cysteines and/or the mutation of a glutamic acid at position 60 described above, and further in addition to the mutation at position 189 and/or position 190 or position 42 and/or position 384 described above.

Further, position 102 in SEQ ID NO: 1 is an amino acid at position 77 in SEQ ID NO: 3, position 379 in SEQ ID NO: 1 is an amino acid at position 354 in SEQ ID NO: 3, and position 447 in SEQ ID NO: 1 is an amino acid at position 422 in SEQ ID NO: 4. In SEQ ID NO: 3 or in an amino acid sequence having an identity of 85%, 90%, or 95% or more with SEQ ID NO: 3, a mutation of one or both of these may be introduced in addition to the mutation in which leucines are substituted with cysteines, the mutation of a glutamic acid at position 60, and/or the mutation of a furin recognition site described above, and further in addition to the mutation at position 189 and/or position 190 or position 42 and/or position 384 described above.

A specific embodiment of the mutant RSV F protein of the present invention is a mutant RSV F protein including an amino acid sequence of any one of SEQ ID NOs: 6 - 9. Specifically, the following amino acid mutations are included.

An amino acid sequence of SEQ ID NO: 6 (a mutant RSV F protein contained in FH_50) is an amino acid sequence in which, in SEQ ID NO: 3, a leucine at position 141 of SEQ ID NO: 1 is substituted with a cysteine, a leucine at position 373 of SEQ ID NO: 1 is substituted with a cysteine, an arginine at position 135 of SEQ ID NO: 1 is substituted with an asparagine, an arginine at position 136 of SEQ ID NO: 1 is substituted with an asparagine, a threonine at position 189 of SEQ ID NO: 1 is substituted with a valine, a serine at position 190 of SEQ ID NO: 1 is substituted with a valine, a proline at position 102 of SEQ ID NO: 1 is substituted with an alanine, an isoleucine at position 379 of SEQ ID NO: 1 is substituted with a valine, and a methionine at position 447 of SEQ ID NO: 1 is substituted with a valine. Here, actually, the amino acid sequence of SEQ ID NO: 6 is divided into an amino acid sequence of an F2 polypeptide and an amino acid sequence of a pep27-added F1 polypeptide (an extracellular region). However, here, they are represented as one amino acid sequence in which they are joined. Therefore, when an identity of amino acid sequence described below is calculated, a comparison is made with an amino acid sequence in which an amino acid sequence of an F2 polypeptide and an amino acid sequence of a pep27-added F1 polypeptide (an extracellular region) of a target protein are joined in this order. The same applies hereinafter.

An amino acid sequence of SEQ ID NO: 7 (a mutant RSV F protein contained in FH_81) is an amino acid sequence in which, in SEQ ID NO: 3, a leucine at position 141 of SEQ ID NO: 1 is substituted with a cysteine, a leucine at position 373 of SEQ ID NO: 1 is substituted with a cysteine, an arginine at position 135 of SEQ ID NO: 1 is substituted with an asparagine, an arginine at position 136 of SEQ ID NO: 1 is substituted with an asparagine, a threonine at position 189 of SEQ ID NO: 1 is substituted with a valine, a serine at position 190 of SEQ ID NO: 1 is substituted with a valine, a glutamic acid at position 60 of SEQ ID NO: 1 is substituted with a methionine, a proline at position 102 of SEQ ID NO: 1 is substituted with an alanine, an isoleucine at position 379 of SEQ ID NO: 1 is substituted with a valine, and a methionine at position 447 of SEQ ID NO: 1 is substituted with a valine.

An amino acid sequence of SEQ ID NO: 8 (a mutant RSV F protein contained in FH_82) is an amino acid sequence in which, in SEQ ID NO: 3, a leucine at position 141 of SEQ ID NO: 1 is substituted with a cysteine, a leucine at position 373 of SEQ ID NO: 1 is substituted with a cysteine, an arginine at position 135 of SEQ ID NO: 1 is substituted with an asparagine, an arginine at position 136 of SEQ ID NO: 1 is substituted with an asparagine, a threonine at position 189 of SEQ ID NO: 1 is substituted with a valine, a serine at position 190 of SEQ ID NO: 1 is substituted with a valine, a lysine at position 42 of SEQ ID NO: 1 is substituted with an arginine, a valine at position 384 of SEQ ID NO: 1 is substituted with a threonine, a proline at position 102 of SEQ ID NO: 1 is substituted with an alanine, an isoleucine at position 379 of SEQ ID NO: 1 is substituted with a valine, and a methionine at position 447 of SEQ ID NO: 1 is substituted with a valine.

An amino acid sequence of SEQ ID NO: 9 (a mutant RSV F protein contained in FH_85) is an amino acid sequence in which, in SEQ ID NO: 3, a leucine at position 141 of SEQ ID NO: 1 is substituted with a cysteine, a leucine at position 373 of SEQ ID NO: 1 is substituted with a cysteine, an arginine at position 135 of SEQ ID NO: 1 is substituted with an asparagine, an arginine at position 136 of SEQ ID NO: 1 is substituted with an asparagine, a threonine at position 189 of SEQ ID NO: 1 is substituted with a valine, a serine at position 190 of SEQ ID NO: 1 is substituted with a valine, a glutamic acid at position 60 of SEQ ID NO: 1 is substituted with a methionine, a lysine at position 42 of SEQ ID NO: 1 is substituted with an arginine, a valine at position 384 of SEQ ID NO: 1 is substituted with a threonine, a proline at position 102 of SEQ ID NO: 1 is substituted with an alanine, an isoleucine at position 379 of SEQ ID NO: 1 is substituted with a valine, and a methionine at position 447 of SEQ ID NO: 1 is substituted with a valine.

One embodiment of the present invention is a mutant RSV F protein having an ability to induce an antibody against an RSV pre-type F protein, comprising the above-described amino acid substitutions in an amino acid sequence having an identity of 85% or more, preferably an identity of 90% or more, and more preferably an identity of 95% or more with an amino acid sequence of any one of SEQ ID NOs: 6 - 9, wherein a disulfide bond is formed between the cysteines.

Definition for an amino acid sequence having a predetermined identity with a predetermined amino acid sequence is the same as that described above.

### (Fusion protein)

Another embodiment of the present invention is a fusion protein that contains the mutant RSV F protein and at least a multimerization domain.

The fusion protein may contain a polypeptide other than the mutant RSV F protein and the multimerization domain. The polypeptide other than the mutant RSV F protein and the multimerization domain is not particularly limited as long as it does not interfere with the effect of the mutant RSV F protein. However, specifically, examples thereof include motifs such as a thrombin cutting motif, and purification tags such as a His-tag or a Strep-Tag II.

The multimerization domain is not particularly limited as long as it is a polypeptide required for the mutant RSV F protein to multimerize. However, a foldon domain for trimerization can be exemplified (Yizhi Tao et al., 1997, Structure 5 (6): 789) (Frank S. et al., 2001, Journal of molecular biology 308 (5): 1081).

As a foldon domain, a foldon domain derived from a bacteriophage T4 fibritin can be exemplified. As an amino acid sequence thereof, the amino acid sequence of SEQ ID NO: 21 can be exemplified.

An example of the fusion protein is a fusion protein in which a foldon domain is linked to a C-terminal of a mutant RSV F protein having an amino acid sequence of SEQ ID NOs: 6 - 9. Specifically, examples thereof include those having amino acid sequences of SEQ ID NOs: 10 - 13.

Actually, the amino acid sequence of SEQ ID NOs: 10 - 13 is divided into an amino acid sequence of an F2 polypeptide and an amino acid sequence of a pep27-added F1 polypeptide (an extracellular region). However, here, they are represented as one amino acid sequence in which they are joined.

### (Multimer)

Another embodiment of the present invention is a multimer of the fusion protein. In the multimer, the fusion protein is associated via a multimerization domain such as a foldon domain. The multimer is preferably a trimer of the fusion protein.

### (Polynucleotide)

Another embodiment of the present invention is a polynucleotide encoding the mutant RSV F protein or the fusion protein.

A polynucleotide encoding the mutant RSV F protein can be obtained as a polynucleotide in which a desired mutation has been introduced using a standard genetic engineering technique with reference to a polynucleotide sequence (SEQ ID NO: 74) encoding the RSV F protein before the introduction of the mutation.

Further, a polynucleotide encoding the fusion protein can also be obtained using a standard genetic engineering technique using a polynucleotide encoding the mutant RSV F protein and a polynucleotide encoding the polypeptide other than the mutant RSV F protein.

Specifically, examples of a polynucleotide sequence to be used in order for the fusion protein of the present invention to be expressed and produced in a host include polynucleotide sequences of SEQ ID NOs: 14 - 17.

SEQ ID NO: 14 is a polynucleotide sequence that is for a fusion protein having the amino acid sequence of SEQ ID NO: 10 to be expressed and produced in a host. However, a sequence encoding a signal peptide is added to a 5' end side of a sequence encoding the fusion protein. SEQ ID NO: 15 is a polynucleotide sequence that is for a fusion protein having the amino acid sequence of SEQ ID NO: 11 to be expressed and produced in a host. However, a sequence encoding a signal peptide is added to a 5' end side of a sequence encoding the fusion protein. SEQ ID NO: 16 is a polynucleotide sequence that is for a fusion protein having the amino acid sequence of SEQ ID NO: 12 to be expressed and produced in a host. However, a sequence encoding a signal peptide is added to a 5' end side of a sequence encoding the fusion protein. SEQ ID NO: 17 is a polynucleotide sequence that is for a fusion protein having the amino acid sequence of SEQ ID NO: 13 to be expressed and produced in a host. However, a sequence encoding a signal peptide is added to a 5' end side of a sequence encoding the fusion protein.

Any codon may be used as long as the codon encodes an intended amino acid. For example, there are four types of codons encoding Gly: GGT, GGC, GGA, and GGG, and any of these can be used. Further, it is possible that each codon is or is not optimized, but it is preferable that each codon is optimized.

The polynucleotide encoding the mutant RSV F protein may be a polynucleotide that hybridizes under a stringent condition to a polynucleotide having a complementary sequence of these polynucleotide sequences, as long as the polynucleotide encoding the mutant RSV F protein encodes a mutant RSV F protein that includes the above specific mutations and has an ability to induce an antibody against an RSV pre-type F protein. Here, an example of the stringent condition is a condition of performing washing at a salt concentration corresponding 68 °C, 0.1 ×SSC, 0.1%SDS after Southern hybridization.

### (Expression unit)

Another embodiment of the present invention is an expression unit that includes the polynucleotide.

The expression unit may include a mechanism for expressing the polynucleotide, for example, may include an element necessary for expressing a promoter sequence, a transcription termination signal sequence, or the like, and may be an expression unit used in a standard genetic engineering technique. The expression unit may be included, for example, in a recombinant vector. Examples of the recombinant vector include a plasmid vector and a viral vector, and examples thereof include a vector that can be expressed in a prokaryotic cell, a vector that can be expressed in an eukaryotic cell, and a vector that can be expressed in a cell derived from a mammal.

### (Host cell)

Another embodiment of the present invention is a host cell that includes the expression unit. Preferably, the host cell is a host cell transformed with a recombinant vector including the expression unit.

The host cell is not particularly limited as long as a protein encoded by the polynucleotide is expressed from the expression unit, and may be used in a standard genetic engineering technique. Specifically, examples thereof include prokaryotic cells such as Escherichia coli and Bacillus subtilis, eukaryotic cells, and mammal-derived cells.

Introduction of the polynucleotide into the host cell can be performed using a commonly known means such as a calcium phosphate method, a DEAE dextran method, an electroporation method, and a lipofection method.

By culturing the host cell under an appropriate condition, the mutant RSV F protein or the fusion protein can be expressed, and the mutant RSV F protein, the fusion protein, or a multimer thereof can be produced. The produced mutant RSV F protein, the fusion protein or the multimer thereof can be purified by a commonly known means.

### (Immunogen)

Another embodiment of the present invention is an immunogen that includes the mutant RSV F protein, the fusion protein, the multimer, or the following particulate product.

That is, the mutant RSV F protein, the fusion protein, the multimer, or the particulate product can be used as an immunogen. The immunogen may include any one of, or any two of, or all of the mutant RSV F protein, the fusion protein, the multimer, or the particulate product as long as the immunogen does not interfere with the effect of the mutant RSV F protein. Among these, the multimer, especially a trimer, is preferable, and the particulate product is preferable, to be used as an immunogen. The production of the multimer is as described above, and the particulate product will be described in detail below. The immunogen of the present invention may include a preservative, an excipient, or the like included in a standard immunogen.

### (Particulate product)

Another embodiment of the present invention is a particulate product of the mutant RSV F protein or the multimer.

The particulate product is not particularly limited as long as it is a particle formed by aggregating two or more mutant RSV F proteins or multimers and can efficiently induce an antibody having a high neutralization capacity. However, for example, the particulate product is a particle formed by aggregating two or more mutant RSV F proteins or multimers via a particle-forming domain. Here, shapes of the particulate product and the particle are not necessarily limited to spherical shapes, and it is sufficient when the aggregation thereof is in a certain direction.

That is, it is preferable that a particle-forming domain is further fused to the mutant RSV F protein or the fusion protein, and a multimer of the mutant RSV F protein or the fusion protein aggregates via the particle-forming domain to form a particle.

It is preferable to perform particle formation using a fusion protein for producing a particulate product in which a particle-forming domain is bound to a C-terminal of the mutant RSV F protein or the fusion protein. The fusion protein for producing a particulate product will be described later.

In a particulate product, the particle-forming domain is preferably located at a position closer to the epitope I than to the epitope Φ on a steric structure of the mutant RSV F protein or the multimer. That is, it is preferably a particulate product in which a core is formed by aggregarion of the particle-forming domain, and the epitope I of the mutant RSV F protein or the multimer exists on an inner side and the epitope Φ is exposed to an outer side.

Therefore, a particulate product according to one embodiment of the present invention is a particulate product in which two or more mutant RSV F proteins or multimers aggregate via a particle-forming domain such that at least the epitope Φ of the RSV F protein is exposed and at least the epitope I of the RSV F protein is not exposed. Here, "the epitope I is not exposed" does not need to mean that the epitope I is completely not exposed as long as the accessibility of antibodies or various immune cells to the epitope I is reduced.

In one embodiment of the particulate product of the present invention, since the epitope Φ and the epitope I of the mutant RSV F protein are located at two extremes on the steric structure of the protein, the particle-forming domain that forms a core is closer to the epitope I than to the epitope Φ, and thereby, a structure can be achieved in which the epitope I which is an unneeded epitope is masked as an antigen and the epitope Φ which is a useful epitope can easily function as an antigen. Here, an unneeded epitope means an epitope that generally induces an antibody having a low RSV viral infection neutralization capacity in humans, when the mutant RSV F protein, a fusion protein thereof, a multimer of these, or a particulate product of theses is used as an immunogen. Specific examples thereof include the epitope I. On the other hand, a useful epitope means an epitope that generally induces an antibody having a high RSV viral infection neutralization capacity in humans, when the mutant RSV F protein, a fusion protein thereof, a multimer of these, or a particulate product of theses is used as an immunogen. Specific examples thereof include the epitope Φ and the epitope V. By adopting this structure, an effect is achieved that the accessibility of antibodies or various immune cells to an unneeded epitope is reduced by a steric hindrance effect.

A particle-forming domain for particle formation is a polypeptide that makes the mutant RSV F protein or its multimer into particles. Examples thereof include those that can bind to scaffolding particles (for example, scaffolding particle-binding peptides), those that can hydrophobically aggregate (for example, hydrophobic peptide chains), those having a self-associating property for example, self-associating peptides), or the like.

First, an embodiment of particle formation using scaffolding particles is described. That is, it is an embodiment in which the mutant RSV F protein or a multimer is bound to scaffolding particles.

A material of the scaffolding particles is not particularly limited. However, examples thereof include proteins having a particle-forming ability, lipid bilayers, envelopes, liposomes, niosomes, virosomes, ferrosomes, gold nanoparticles, resin, silica, polymer micelles, gels, and the like.

Examples of proteins having a particle-forming ability include viral proteins or variants of viral proteins that form VLPs (virus-like particles) or VPs (virus particles) (for example, Japanese Patent Application Laid-Open Publication No. 2004-2313). For example, a hepatitis B virus surface antigen protein modified to delete an original infectious ability with respect to hepatocytes and further modified to present a scaffolding molecule can be mentioned, and, when expressed in eukaryotic cells, this protein is expressed and accumulated as a membrane protein on a membrane such as an endoplasmic reticulum membrane, and is released as a VLP.

As scaffolding particles, more specifically, protein-derived VLPs produced by inserting an Fc binding domain (Z domain) of a protein A in a PreS region of a hepatitis B virus surface antigen (HBs antigen) can be mentioned, and, for example, Bionanocapsule-ZZ (Beacle catalog number: BCL-DC-002) can be used.

It is preferable that a molecule (scaffolding molecule) that binds to a scaffolding particle-binding peptide as a particle-forming domain is immobilized on a surface of a scaffolding particle.

For example, an Fc binding domain of a protein A can be mentioned, and the mutant RSV F protein or a multimer thereof can be bound via a scaffolding particle-binding peptide.

A bond between a scaffolding molecule and a scaffolding particle-binding peptide may be either a covalent bond or a non-covalent bond. Examples of covalent bonds include methods collectively referred to as click chemistry (an alkyne (such as acetylene)-azide bond, a cysteine-maleimide bond, and a primary amine-NHS ester bond are exemplified), a method for generating a charge shift bond (a hydrazide-aldehyde bond is exemplified), a method for generating a covalent bond depending on a polypeptide (such as a method using SpyTag-SpyCatcher), and the like. Examples of non-covalent bonds include an ionic interaction, a hydrophobic bond, a hydrogen bond, and the like.

Specifically, the examples include combinations of protein tags and protein tag-binding molecules (biotin-avidin, Fc-protein A, GST (glutathione S-transferase)-glutathione, MBP (maltose binding protein)-maltose, His Tag-nickel, and SBP Tag-streptavidin are exemplified), a combination of an antibody and an antigen, and the like.

A preferred combination of a scaffolding molecule and a scaffolding particle-binding peptide is a combination of an Fc domain of IgG1 (SEQ ID NO: 30) and an Fc-binding domain (Z domain) of a protein A. An Fc domain of IgG1 may be used for a scaffolding molecule and an Fc binding domain of a protein A may be used for a scaffolding particle-binding peptide. An Fc domain of IgG1 may be used for a scaffolding particle-binding peptide, and an Fc-binding domain of a protein A may be used for a scaffolding molecule.

As long as the above scaffolding molecules or scaffolding particle-binding peptides can be covalently or non-covalently bound to each other, partial fragments thereof can be used or modified.

Next, an embodiment of particle formation using a hydrophobic peptide chain as a particle-forming domain is described.

In this embodiment, a hydrophobic peptide chain is bound to the mutant RSV F protein or the fusion protein, and the mutant RSV F protein or the fusion protein forms a particle via the hydrophobic peptide chain.

A hydrophobic peptide chain means a peptide chain that contains a hydrophobic amino acid and self-assembles to form the particulate product, and that contains 5 - 50, preferably 10 - 30, amino acid residues. Here, examples of highly hydrophobic amino acids include valine, leucine, phenylalanine, isoleucine and the like, and leucine or isoleucine is more preferable. A ratio of hydrophobic amino acids in a hydrophobic peptide chain is preferably 20 - 60%.

In a hydrophobic peptide chain, it is preferable that hydrophobic amino acids appear on a surface of the hydrophobic peptide chain. A ratio of the hydrophobic amino acids on the surface is preferably 10% or more. However, when the ratio is too high, it aggregates inside cells and is not secreted, and thus, the hydrophobic amino acids on the surface are preferably 50% or less, 30% or less, and 20% or less. On the other hand, a higher ratio of hydrophobic amino acids on an inner side leads to a stable structure of the hydrophobic peptide chain, and thus is preferable.

Any amino acid other than a hydrophobic amino acid may be used. However, in particular, in order to enhance a secretory effect, it is preferable to contain 20% or more of histidine.

By forming a hydrophobic peptide chain into a repeating sequence of a peptide consisting of 7 amino acids, a helix structure is formed and the above design can be easily performed, and thus, it is preferably used. For example, a hydrophobic peptide chain is preferably a repeating sequence of a peptide sequence consisting of 7 amino acids in which hydrophobic amino acids are arranged at positions 1, 3 and 5. The number of repetitions is, for example, 2 - 5, and preferably 2 - 3.

Preferred examples of the hydrophobic peptide chain include CC02, CC03, CC07, and CC08 (in this order, SEQ ID NOs: 40, 41, 42, and 43), and, among these, CC07 (SEQ ID NO: 42) is more preferable.

Next, an embodiment of particle formation using a self-associating protein domain as a particle-forming domain is described.

In this embodiment, a self-associating protein domain is bound to the mutant RSV F protein or the fusion protein, and the mutant RSV F protein or the fusion protein forms a particle via the self-associating protein domain.

Examples of self-associating proteins include a hepatitis B virus core antigen (HBcAg), a human papillomavirus capsid protein (HPV L1), a hepatitis E virus capsid protein (HEV capsid), a bacteriophage Qβ coat protein, a norwalk virus (Norovirus, NoV) capsid, a parvovirus B19 capsid, an alfalfa mosaic virus, feritin, encapsulin, and the like. A self-associating protein may be a self-associating partial fragment or a variant.

In a case where HBcAg is used, when binding to the RSV F protein or the multimer of the present invention, it is preferable to bind the RSV F protein or the multimer to a site far from an associating surface for self-association of HBcAg and on an outer side of those self-associated. Further, when such binding is performed, it is preferable to insert a linker between a helix of an α3 domain and a helix of an α4 domain in order to obtain an original steric structure of HBcAg. The linker is, for example, a polypeptide having 10 - 30 amino acids, and is, for example, a GS linker. An example of a sequence in which a FLAG tag is bound to such a variant of HBcAg is SEQ ID NO: 54.

### <Fusion protein for producing the particulate product>

A fusion protein for producing the particulate product is a protein used for producing the above particulate product, and contains a particle-forming domain, such as the scaffolding particle-binding peptide, the hydrophobic peptide chain, or the self-associating protein domain described above, which is fused to a C-terminal of the mutant RSV F protein of the present invention or a C-terminal of the fusion protein.

The present fusion protein for producing a particulate product aggregates via the particle-forming domain to form a particulate product.

When a distance between C-terminals of fusion proteins that form the multimer of the present invention is changed due to binding to the particle-forming domain, by inserting a linker between the particle-forming domain and the C-terminals (multimerization domain), the influence on the distance between the C-terminals can be reduced. The linker is not particularly limited, but is preferably a peptide having 5 - 20 amino acids, and more preferably a peptide having 5 - 10 amino acids. The sequence is not particularly limited as long as it does not inhibit a vaccine effect of the fusion protein, but is, for example, a GS linker consisting of glycine and serine.

The position of the linker may be between the multimerization domain and the particle-forming domain of the mutant RSV F protein or the fusion protein, and tag sequences may be included before and after the linker. Tag sequences are not particularly limited. However, tag sequences commonly used for protein purification are preferably used. For example, a FLAG tag (SEQ ID NO: 28), a His-tag (SEQ ID NO: 25), a Strep-tag II (SEQ ID NO: 26), and the like can be exemplified.

The fusion protein for producing a particulate product can be obtained by linking, in accordance with a reading frame, a DNA encoding the particle-forming domain, and further when necessary, a DNA encoding a linker or a tag, to a DNA encoding the mutant RSV F protein or the fusion protein, and expressing it in a host. An expression and purification method can be performed in the same manner as the expression and purification of the mutant RSV F protein or the fusion protein.

Specific examples of the fusion protein for producing a particulate product include an embodiment in which a scaffolding particle-binding peptide is bound to a mutant RSV F protein (sequences of precursor proteins thereof: SEQ ID NOs: 31 - 39, and polynucleotide sequences encoding it: SEQ ID NOs: 57 - 65), an embodiment in which a hydrophobic peptide chain is bound to a mutant RSV F protein (sequences of precursor proteins thereof: SEQ ID NOs: 44 - 49, and polynucleotide sequences encoding it: SEQ ID NOs: 66 - 71), and an embodiment in which a self-associating protein domain is bound to a mutant RSV F protein (sequences of precursor proteins thereof: SEQ ID NOs: 55 - 56, or polynucleotide sequences encoding it: SEQ ID NOs: 72 - 73).

The fusion protein for producing a particulate product may contain tag sequences such as a FLAG tag (DYKDDDDK: SEQ ID NO: 28) and a tag (GGSDYKDDDDK: SEQ ID NO: 29) in which 3 amino acids (GGS) are added to the FLAG tag.

### (Method for producing particulate product)

A particulate product can be obtained by performing particle formation using the above-obtained fusion protein for producing a particulate product, and then purifying the particulate product when necessary.

When scaffolding particles are used, the particle formation step can be performed by mixing fusion proteins for producing particulate products with the scaffolding particles, and then, removing mutant RSV F proteins or its products that do not form particles and purifying the particulate products. As the scaffolding particles, for example, HBsAg VLP (Beacle Catalog No: BCL-DC-002) can be used.

When fusion proteins for producing the particulate products containing hydrophobic peptide chains are used, the particle formation step can be performed by associating the fusion proteins for producing particulate products via the hydrophobic peptide chains to form particulate products, and then, removing mutant RSV F proteins or its products that do not form particles and purifying the particulate products.

When fusion proteins for producing particulate products containing self-associating protein domains are used, the particle formation step can be performed by associating the fusion proteins for producing particulate products via the self-associating protein domains to form particulate products, and then, removing mutant RSV F proteins or its products that do not form particles and purifying the particulate products.

In the particulate products of the present invention, it is desirable that, per one particulate product, for example, 2 or more, preferably 10 or more, and more preferably 20 or more trimers are bound.

Further, in the particulate products of the present invention, it is desirable that, per 1 nm² of a particulate product surface area, for example, 0.0001 or more, preferably 0.0005 or more, and more preferably 0.001 or more trimers are bound. Here, the particulate product surface area means a surface area when a particulate product is assumed to be a sphere of which a diameter is a theoretical diameter measured using a dynamic light scattering method. The number per particulate product surface area can be calculated, for example, using a method in which the number per one particulate product is divided by the particulate product surface area.

By producing particulate products and selecting those having a low binding ability with respect to the epitope I and a high binding ability with respect to the epitope Φ, a person skilled in the art can select an optimal number per one particulate product or an optimal number per 1 nm² of the particulate product surface area.

In the present invention, a RSV F protein or a multimer used in particle formation is not limited to the mutant RSV F protein or the multimer of the present invention, and is not particularly limited as long as it can be used as a RSV vaccine, and a mutant RSV F protein other than the mutant RSV F protein of the present invention or a multimer thereof can also be used. For example, those described in International Publication No. 2017/172890 or International Publication No. 2017/109629 may be used. That is, the particle formation technology of the present invention can be generally used in particle formation of RSV F proteins or multimers thereof. The particulate products of the present invention are particulate products of RSV F proteins or multimers thereof, and include particulate products formed by aggregating two or more RSV F proteins or multimers thereof via particle-forming domains. As the particle-forming domains and the method of particle formation, the particle-forming domains and the particle formation method described above in the particle formation of the mutant RSV F proteins or the multimers thereof can be applied.

### (Pharmaceutical composition)

Another embodiment of the present invention is a pharmaceutical composition that includes the expression unit or the immunogen as an active ingredient.

The expression unit or the immunogen can be a pharmaceutical composition for preventing or treating RS virus infection.

Another embodiment of the present invention is an RSV vaccine that includes the expression unit or the immunogen. This is also an embodiment in which the pharmaceutical composition according to the above embodiment is an RSV vaccine.

The pharmaceutical composition (including the RSV vaccine, the same applies hereinafter) is not particularly limited in route of administration, and can be administered to mammals by either parenteral administration (for example, intradermal, intramuscular, subcutaneous, transdermal, and mucosal administered) or oral administration. Examples of mammals include humans, mice, rats, rabbits, dogs, cats, cows, pigs, monkeys, chimpanzees, and the like, but humans are preferred.

A dosage form of the pharmaceutical composition and a preparation method thereof are commonly known to a person skilled in the art, and the pharmaceutical composition can be produced by blending the expression unit or the immunogen with a pharmaceutically acceptable carrier or the like.

Examples of the pharmaceutically acceptable carrier include an excipient, a lubricant, a binding agent and a disintegrating agent in a solid formulation, or a solvent, a solubilizing agent, a suspending agent, an isotonizing agent, a buffering agent, a soothing agent and the like in a liquid formulation. Further, when necessary, additives such as an ordinary antiseptic agent, an anti-oxidizing agent, a coloring agent, a sweetener, an adsorbent, and a wetting agent can be appropriately used in appropriate amounts.

The pharmaceutical composition may include an adjuvant. Examples of the adjuvant include a gel type, a bacterial cell type, an oil emulsion type, a polymer nanoparticle type, a synthetic type, a cytokine type, and the like.

Examples of the gel type include aluminum hydroxide, aluminum phosphate, calcium phosphate and the like.

Examples of the bacterial cell type include CpG, cholera toxin, Escherichia coli thermophilic toxin, pertussis toxin, Muramyl dipeptide (MDP), and the like.

Examples of the oil emulsion type include Freund's incomplete adjuvant, MF59, SAF, and the like.

Examples of the polymer nanoparticle type include immunostimulatory complexes, liposomes, biodegradable microspheres, saponin-derived QS-21, and the like.

Examples of the synthetic type include nonionic block copolymers, muramyl peptide analogs, polyphosphazene, synthetic polynucleotides, and the like.

Examples of the cytokine type include IFN-γ, IL-2, IL-12, and the like.

Examples of the dosage form for parenteral administration include injection formulations (such as drip injection, intravenous injection, intramuscular injection, subcutaneous injection, intradermal injection, intracerebral administration formulation, and intraspinal administration formulation), external preparations (such as ointment, poultice, and lotion), suppository inhalants, ophthalmic agents, eye ointments, nasal drops, ear drops, liposomal agents, and the like.

Examples of the dosage form for oral administration include solid or liquid dosage forms, and, specifically, include tablets, coated tablets, pills, fine granules, granules, powders, capsules, syrups, emulsions, suspensions, troches, and the like.

The pharmaceutical composition of the present invention is preferably a liquid dosage form, and preferably an injection.

An effective dose of the pharmaceutical composition is determined, for example, by a physician based on various factors such as a route of administration, a type of disease, severity of symptoms, age, gender and body weight of a patient, severity of disease, pharmacological findings such as pharmacokinetics and toxicological features, whether or not a drug delivery system is used, and whether or not the pharmaceutical composition is administered as part of a combination with other drugs.

### [Examples]

Examples are described below. However, none of the examples is to be interpreted in a limited manner.

### [Example 1]

The formation of a covalent bond (disulfide bond) using cysteines is useful for stabilizing a structure of a protein. In previous studies, various disulfide mutations that stabilize a pre-F structure in the RSV F protein (hereinafter, may be referred to as an "F protein") have been reported (International Publication No. 2017/172890, and International Publication No. 2017/109629). However, since an amino acid that is member of a secondary structure is substituted with a cysteine, or a disulfide is formed at a distant part, there is a high risk that a resulting mutant may have a structure different from a natural structure (a pre-fusion structure that a natural F protein is thought to have during protein expression). Therefore, it is decided to stabilize a structure by substituting, with cysteines, an amino acid pair that does not have a secondary structure and has a distance between S atoms shorter than 5 Å when being substituted with cysteines. In calculation of a distance, a published structural model (PDB registration number: 4MMS) was used and two arbitrary amino acids were substituted with cysteines to generate a conformation having an enegetically stable dihedral angle N-Ca-Cb-S, and a shortest distance between S atoms was calculated (Table 1). As a result, mutation sites of the mutant RSV F protein (which may be referred to as a "mutant protein" in the present specification) contained in antigens FH 08 and FH_09 to be described later were listed as candidates. The two candidates do not have a polar functional group in a side chain of an amino acid before mutation at a mutation site, and thus, were thought to be excellent also in that there is no risk of structural instability due to loss of a polar group interaction (a hydrogen bond or the like) present in a natural F protein. "L141C" in Table 1 indicates that a leucine at position 141 in the amino acid sequence of SEQ ID NO: 1 has mutated to a cysteine. Hereinafter, the same notation is used in the table. A precursor protein (SEQ ID NO: 4) of an F_native antigen is a polypeptide in which, in a polypeptide (amino acid numbers 1 - 513 of SEQ ID NO: 1) obtained by removing a transmembrane region and an intracellular region of an F1 polypeptide from a natural F0 protein of an A2 strain, a foldon domain (SEQ ID NO: 21), and a sequence (SEQ ID NO: 22) including a Thrombin recognition sequence, a His tag, and a StrepTag II are sequentially fused to a C-terminal side of a polypeptide having mutations of P102A, I379V, and M447V. In the present example, mutants were all created based on the precursor protein of the F_native antigen, and thus have the mutations of P102A, I379V, and M447V. The F_native precursor protein is subjected to processing during an expression process, and a signal peptide and a pep27 region fall off.

### [Table 1]

**Table 1**

| Antigen name | Mutation content | Structural analysis result | |
|---|---|---|---|
| | | Distance between S atoms (Å) | Secondary structure |
| F_native | None | - | |
| FH_08 | L141C, L373C | 4.35 | None |
| FH_09 | L142C, L373C | 4.72 | None |

The F_native antigen is produced by expressing a polynucleotide (SEQ ID NO: 5) encoding a precursor protein (SEQ ID NO: 4) of the F_native antigen using a mammalian cell as a host. The F_native antigen forms a quaternary structure after the precursor protein (SEQ ID NO: 4) of the F_native antigen is subjected to processing during the expression process. Due to the processing, the signal peptide and the pep27 region fall off, and the remaining F2 polypeptide sequence and a sequence including the F1 polypeptide (extracellular region), a foldon domain, a Thrombin recognition sequence, a His tag, and a Strep-Tag II are bound by a disulfide bond, and further, a homo trimer is expected to be formed by a binding force mainly generated by the foldon domain. A specific production method of the F native antigen is shown below. The above polynucleotide (SEQ ID NO: 5) was inserted into a pcDNA3.4 vector (Thermo Fisher Scientific, Inc.) using a standard genetic engineering technique. That a target DNA sequence has been inserted was confirmed by a base sequence analysis, and a mammalian cell expression vector expressing the precursor protein of the F_native antigen was constructed. A vector for introduction into a cell was purified using a standard plasmid vector purification method.

Expression of the F_native antigen was carried out by mammalian cell secretion expression using an Expi293 Expression System (Thermo Fisher Scientific, Inc.). The purified vector was introduced into an Expi293 cell using an Expi Fectamin 293 Reagent, which is a gene transfer reagent of the same kit, the encoded F_native antigen was secreted and expressed by culturing for about 5 days, and only a supernatant component was obtained by centrifugation and using a filter with a hole diameter of 0.22 µm.

Purification of the F_native antigen was carried out by affinity chromatography using Ni Sepharose High Performance (GE Healthcare, Inc.). A Ni carrier was caused to react with the above culture supernatant component diluted about 3 times with a binding buffer (20 mmol/L Tris-HCl pH 7.4, 500 mmol/L NaCl, 20 mmol/L imidazole) for a whole day and night, and then, it was applied to an elution buffer (20 mmol/L Tris-HCl pH 7.4, 500 mmol/L NaCl, 500 mmol/L imidazole) to elute each antigen. After that, solvent substitution with PBS (pH 7.4) was performed using an ultrafiltration membrane, and it was used as the F_native antigen.

A polynucleotide encoding a precursor protein of the antigen FH_08, in which a double mutation of L141C and L373C was introduced into the precursor protein of the F_native antigen, was prepared in the same manner as above. Further, a polynucleotide encoding a precursor protein of the antigen FH_09, in which a double mutation of L142C and L373C was introduced into the precursor protein of the F_native antigen, was prepared in the same manner as above. These were expressed and purified using the above method to produce the antigens FH_08 and FH_09.

A binding property of each antigen to each antibody was measured using Octet QK384 (ForteBio, Inc.) under conditions of PBS (pH 7.4), 25 °C, and 1000 rpm. Each antibody was bound to a sensor chip by applying each antibody solution for 180 seconds to a Protein A sensor chip (ForteBio, Inc.) equilibrated with PBS (pH 7.4), and an amount of increase in a detected value when each antigen solution was subsequently applied for 180 seconds was defined as a value of a binding signal of each antigen to each antibody. As an antibody applied to a sensor chip, a D25 antibody capable of specifically detecting the epitope Φ was used. A value of a binding signal was normalized by determining a ratio with respect to a value of a binding signal when Palivizumab (a Synagis intramuscular injection, AbbVie, Inc.) capable of binding regardless of a conformational change of an F antigen, was used.

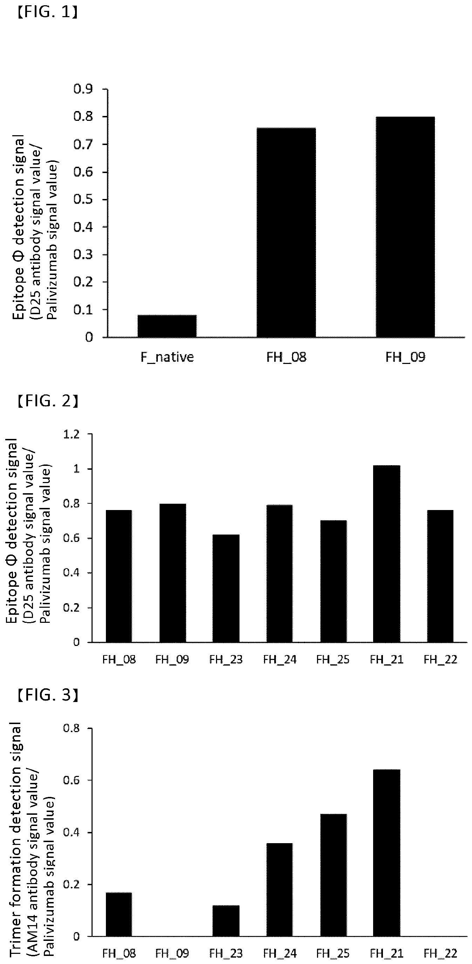

By the above operation, a result was obtained indicating that the antigens FH_08 and FH_09 containing the mutant proteins in which cysteine point mutations were introduced at two places for disulfide bond formation have significantly improved the storage stability of the epitope Φ as compared to the F_native antigen containing the F protein before the introduction of the mutations (Fig. 1).

### [Example 2]

There is a report that a natural F protein is first cleaved at a first furin recognition site to form a monomer structure, and then is cleaved at a second furin recognition site to eliminate a pep27 region, thereby promoting trimerization (Anders Krarup et al., 2015, Nature communications 6:8143). However, since the pep27 region is highly conserved in sequence length and amino acid type among virus strains isolated from nature, it is thought that the pep27 region may have an indispensable effect on formation of a steric structure of an antigen. Therefore, a mutation was introduced into the second furin recognition site to inhibit cleavage of the pep27 region and an effect of the mutation on trimer formation was investigated. Mutation contents of precursor proteins of antigens prepared below are summarized in Table 2. "FH_08 + R135N, R136N" in Table 2 indicates that, in the precursor protein of the antigen FH 08, further, an arginine at position 135 in the amino acid sequence of SEQ ID NO: 1 is mutated to an asparagine and an arginine at position 136 is mutated to an asparagine. "FH_09 + R135N, R136N" indicates that, in the precursor protein of the antigen FH_09, further, an arginine at position 135 in the amino acid sequence of SEQ ID NO: 1 is mutated to an asparagine and an arginine at position 136 is mutated to an asparagine. In the antigens FH23 - 25, a pep27 (p27) sequence in the precursor protein of the antigen FH08 was substituted with various GS linkers. As the GS linkers, GSGSGS (SEQ ID NO: 18), (GGGGS)₂ (SEQ ID NO: 19), (GGGGS)₃ (SEQ ID NO: 20) were used.

### [Table 2]

**Table 2**

| Antigen name | Mutation content |
|---|---|
| FH_08 | L141C, L373C |
| FH_09 | L142C, L373C |
| FH_23 | Substitute p27 sequence of FH_08 with GSGSGS (SEQ ID NO: 18) |
| FH_24 | Substitute p27 sequence of FH_08 with (GGGGS)₂ (SEQ ID NO: 19) |
| FH_25 | Substitute p27 sequence of FH_08 with (GGGGS)₃ (SEQ ID NO: 20) |
| FH_21 | FH_08 + R135N, R136N |
| FH 22 | FH_09 + R135N, R136N |

A polynucleotide encoding each of the precursor proteins of the antigens was inserted into a pcDNA3.4 vector (Thermo Fisher Scientific, Inc.) using a standard genetic engineering technique. That a target DNA sequence has been inserted was confirmed by a base sequence analysis, and mammalian cell expression vectors expressing the precursor proteins of the antigens were each constructed. A vector for introduction into a cell was purified using a standard plasmid vector purification method.

Expression and purification of each antigen were carried out by the same operation as in Example 1.

A binding property of each antigen to each antibody was also measured by the same operation as in Example 1. However, an AM14 antibody capable of detecting trimer formation of a pre-F was added as an antibody to be applied to a sensor chip. A value of a binding signal of the D25 antibody and the AM14 antibody was normalized by determining a ratio with respect to a value of a binding signal when a Synagis antibody (AbbVie, Inc., a Synagis intramuscular injection) capable of binding regardless of a conformational change of an F antigen, was used.

By the above operation, a result was obtained indicating that the antigens FH_21, FH_24, and FH_25 containing the mutant proteins in each of which a mutation was added to the second furin recognition site have improved trimer formation while maintaining the storage stability of the epitope Φ as compared to the antigen FH_08 before the addition of the mutation. Among them, the improvement in trimer formation was prominent in the antigen FH 21, which is closer to the pep27 region of the F_native antigen, as compared to those (FH_24 and FH_25) in which the pep27 region was substituted with an artificial linker (Figs. 2 and 3).

### [Example 3]

Amino acids at position 189 and position 190 of the precursor protein of the F_native antigen are a threonine and a serine, and are hydrophilic amino acids in that they each have a hydroxyl group in a side chain. However, amino acids located sterically proximal to the amino acids at positions 189 - 190 are an isoleucine at position 57, an isoleucine at position 167, a leucine at position 171, a valine at position 179, and a leucine at position 260, and the like. However, since all of these are hydrophobic amino acids, it is thought there is a possibility that the structural stability may be impaired. Therefore, a possibility of structural stabilization and contributing to an improvement in expression level by mutating the amino acids at positions 189 - 190 to hydrophobic amino acids (valine, leucine, isoleucine) was considered. Mutation contents of precursor proteins of antigens prepared below are summarized in Table 3. "FH_21 + T189I" in Table 3 indicates that, in the precursor protein of the antigen FH 21, further, a threonine at position 189 in the amino acid sequence of SEQ ID NO: 1 is mutated to an isoleucine. "FH_21 + T189V" indicates that, in the precursor protein of the antigen FH 21, further, a threonine at position 189 in the amino acid sequence of SEQ ID NO: 1 is mutated to a valine. "FH_21 + T189I, S190V" indicates that, in the precursor protein of the antigen FH_21, further, a threonine at position 189 in the amino acid sequence of SEQ ID NO: 1 is mutated to a isoleucine, and a serine at position 190 is mutated to a valine. "FH_21 + T189L, S190V" indicates that, in the precursor protein of the antigen FH_21, further, a threonine at position 189 in the amino acid sequence of SEQ ID NO: 1 is mutated to a leucine, and a serine at position 190 is mutated to a valine. "FH_21 + T189V, S190V" indicates that, in the precursor protein of the antigen FH 21, further, a threonine at position 189 in the amino acid sequence of SEQ ID NO: 1 is mutated to a valine, and a serine at position 190 is mutated to a valine. "FH_50 + K42R, V384T" indicates that, in the precursor protein of the antigen FH_50, further, a lysine at position 42 in the amino acid sequence of SEQ ID NO: 1 is mutated to an arginine, and a valine at position 384 is mutated to a threonine. Further, point mutations occurring in natural virus isolates were extracted and individual mutations were each examined. An effect was confirmed that an expression level was improved at two point mutations (K42R, V384T), and thus, examination was performed by introducing also this mutation.

### [Table 3]

**Table 3**

| Antigen name | Mutation content |
|---|---|
| FH_21 | L141C, L373C, R135N, R136N |
| FH_55 | FH_21 + T189I |
| FH_53 | FH_21 + T189V |
| FH_52 | FH_21 + T1891, S190V |
| FH_51 | FH_21 + T189L, S190V |
| FH_50 | FH_21 + T189V, S190V |
| FH_82 | FH_50 + K42R, V384T |

A polynucleotide encoding each of the precursor proteins of the antigens was inserted into a pcDNA3.4 vector (Thermo Fisher Scientific, Inc.) using a standard genetic engineering technique. That a target DNA sequence has been inserted was confirmed by a base sequence analysis, and mammalian cell expression vectors expressing the precursor proteins of the antigens were each constructed. A vector for introduction into a cell was purified using a standard plasmid vector purification method.

Expression and purification of each the antigens were carried out by the same operation as in Example 2.

An expression level of each antigen was measured by an absorbance measurement using a standard spectrophotometer. An expression level y (y = A280xM/εxVp/Vc) per expression culture volume was calculated using an absorbance (280 nm) A280 of a purified antigen solution, a molar absorbance coefficient ε and a molecular weight M which were estimated from amino acid primary sequence information, an antigen solution volume Vp after purification, and an expression culture volume Vc.

A binding property of each antigen to the D25 antibody and the AM14 antibody was measured by the same operation as in Example 2.

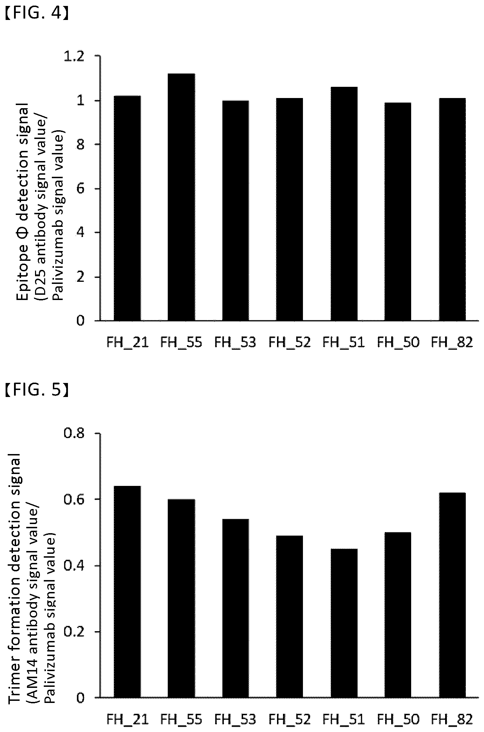

By the above operation, a result was obtained showing that the antigens FH_50, FH_51, FH_52, FH 53, and FH_55 in each of which a mutation was added to the hydrophobic amino acids (V, I, L) at positions 189 - 190 have significantly improved an expression level while maintaining the storage stability of the epitope Φ and the trimer formation as compared to the antigen FH_21 before the addition of the mutation. Further, a result was obtained showing that the antigen FH_82 in which the two point mutations (K42R, V384T) extracted from natural mutations that can occur in natural viruses were added has further significantly improved an expression level while maintaining the storage stability of the epitope Φ and the trimer formation (Figs. 4 - 6).

### [Example 4]

An amino acid at position 60 of the precursor protein of the F_native is a glutamic acid and belongs to an acidic amino acid, and thus, is generally known to be negatively charged under a condition in which a solvent is neutral. This amino acid is sterically adjacent to an aspartic acid at position 194 which is also negatively charged under a neutral condition, and a possibility of a structural instability due to proximity of negative charges was considered. Therefore, in the amino acid sequence of the precursor protein of the F_native, a mutation was carried out to change the amino acid at position 60 to an amino acid other than an acidic amino acid. However, when changing the amino acid at position 60 to an asparagine, in order to prevent an amino acid at positions 60 - 62 from becoming a commonly known N-type sugar chain binding motif (Asn-X-Ser/Thr), an amino acid at position 62 was changed at the same time from a serine to an alanine. Mutation contents of precursor proteins of antigens prepared below are summarized in Table 4.

### [Table 4]

**Table 4**

| Antigen name | Mutation content |
|---|---|
| F_native | None |
| FH_124 | E60R |
| FH_125 | E60M |
| FH_126 | E60F |
| FH_57 | E60A |
| FH_58 | E60H |
| FH_59 | E60I |
| FH_60 | E60L |
| FH_61 | E60N, S62A |
| FH_62 | E60Q |
| FH_63 | E60S |
| FH_64 | E60T |
| FH_65 | E60V |
| FH_66 | E60W |
| FH_67 | E60Y |

A polynucleotide encoding each of the precursor proteins of the antigens was inserted into a pcDNA3.4 vector (Thermo Fisher Scientific, Inc.) using a standard genetic engineering technique. That a target DNA sequence has been inserted was confirmed by a base sequence analysis, and mammalian cell expression vectors expressing the precursor proteins of the antigens were each constructed. A vector for introduction into a cell was purified using a standard plasmid vector purification method.

Expression and purification of each the antigens were carried out by the same operation as in Example 2.

A binding property of each antigen to the D25 antibody and the AM14 antibody was measured by the same operation as in Example 2.

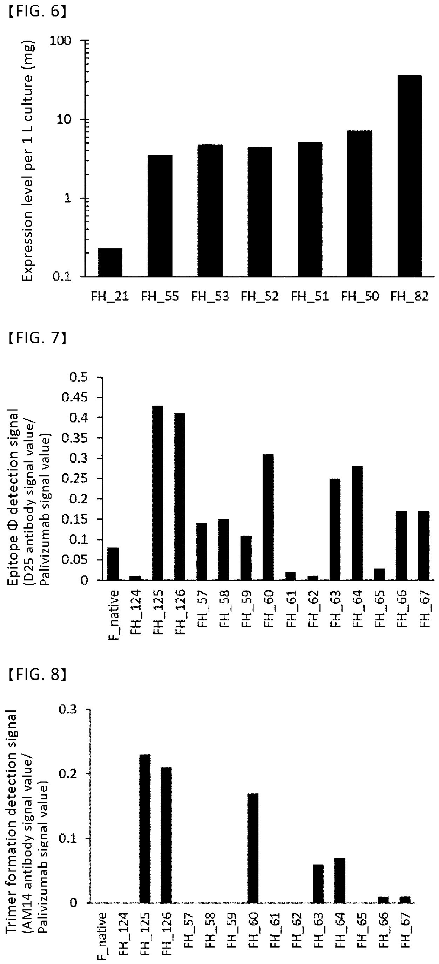

By the above operation, a result was obtained showing that the storage stability of the epitope Φ and the trimer formation were improved when position 60 was changed to M, F, L, S, and T (Figs. 7 and 8).

### [Example 5] Comparison with predecessor

One of most well-reported mutations as a technique for stabilizing a pre-F of an F antigen is a DS-Cavl mutation (S155C, S290C, S190F, V207L) (Jason S. McLellan et al., 2013, Science 342: 592). Therefore, the antigens shown in Table 5 in which useful mutations were further introduced into the useful antigen FH 50 and antigen FH 50 found in the above-described examples were compared to the antigen F_DS-Cav1 into which the DS-Cavl mutation was introduced. Further, an antigen ΔFP furin_{wt} F_{ecto} (Kurt A. Swanson et al., 2014, Journal of Virology, 88 (20): 11802), which is known to have a post-F trimer structure, was also prepared, and was used for evaluation of the antigens. Mutation contents of precursor proteins of antigens prepared below are summarized in Table 5. The precursor proteins of the antigens FH_81 - 85 were each obtained by introducing a mutation of E60M, K42R + V384T or E60M + K42R + V384T into the precursor protein of the antigen FH_50.

### [Table 5]

**Table 5**

| Antigen name | Mutation content |
|---|---|
| F_DS-Cav1 | S155C, S290C, S190F, V207L |
| FH_50 | L141C, L373C, R135N, R136N, T189V, S190V |
| FH_81 | FH_50 + E60M |
| FH_82 | FH_50 + K42R, V384T |
| FH_85 | FH_50 + E60M, K42R, V384T |

A polynucleotide encoding each of the precursor proteins of the antigens was inserted into a pcDNA3.4 vector (Thermo Fisher Scientific, Inc.) using a standard genetic engineering technique. The precursor protein of the antigen F_DS-Cav1 was an amino acid sequence consisting of SEQ ID NO: 23, and the precursor protein of ΔFP furin_{wt} F_{ecto} was an amino acid sequence consisting of SEQ ID NO: 24. That a target DNA sequence has been inserted was confirmed by a base sequence analysis, and mammalian cell expression vectors expressing the precursor proteins of the antigens were each constructed. A vector for introduction into a cell was purified using a standard plasmid vector purification method.

Expression and purification of each of the antigens were carried out by the same operation as in Example 2.

An expression level of each antigen was carried out by the same operation as in Example 3.

### (Antibody binding property test)

A binding property of each antigen to each antibody was measured by the same operation as in Example 2. However, a 131-2A antibody that recognizes the epitope I was added as an antibody to be applied to a sensor chip. A value of a binding signal of the D25 antibody, the AM14 antibody and the 131-2A antibody was normalized by determining a ratio with respect to a value of a binding signal when a Synagis antibody (AbbVie, Inc., a Synagis intramuscular injection) capable of binding regardless of a conformational change of an F antigen, was used.

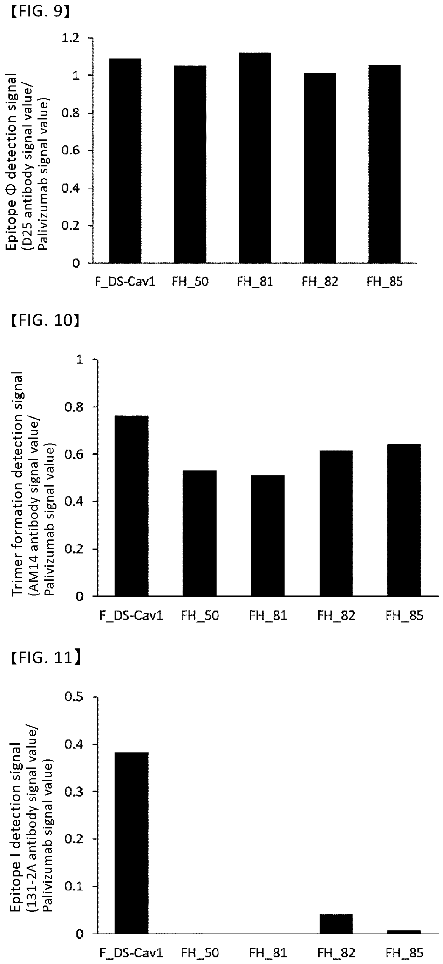

By the above operation, a result was obtained showing that, for the antigens FH_50, FH_81, FH_82, and FH_85, as compared to the antigen F_DS-Cav1 in the prior art document, while there is no significant change in the storage stability of the epitope Φ and the trimer formation, the epitope I, which is known to induce an antibody having significantly low viral infection neutralization capacity, is hardly detected (Figs. 9 - 11). This suggests that although a vaccine carries a risk of exacerbating symptoms by activating an immune cell that contributes little to virus neutralization, the risk is lower for the introduction of the newly found mutations as compared to the introduction of the DS-Cavl mutation.

### (Adsorption test of virus neutralizing antibody in human serum)

By the above operation, it was shown that there is a possibility that the antigens FH_50, FH_81, FH 82, FH_85 have an effect of suppressing induction of an immunity that does not contribute to virus neutralization. In order to investigate the effect in more detail, using healthy adult serums, what kinds of antibodies in the healthy adult serums the antigens FH 50, FH 81, FH 82, FH_85 and F_DS-Cav1 bind to was verified.

Three healthy adult serums were subjected to antigen adsorption operations using magnetic beads Dynabeads His-Tag Isolation and Pulldown (Thermo Fisher Scientific, 10103D). First, 20 µg of the antigen F_DS-Cav1, FH_50, FH_81, FH_82 or FH_85 was applied to and caused to be bound to beads of 45 µL of a bead solution washed twice with PBS (pH 7.4), and then, blocking with bovine serum albumin was performed. Next, 100 µL of each healthy adult serum was diluted 10-fold with PBS (pH 7.4), the beads were added thereto, and the mixture was incubated (4 °C, 1 hour, rotated), and then, removal of antibodies bound to antigens was performed by removing the beads.

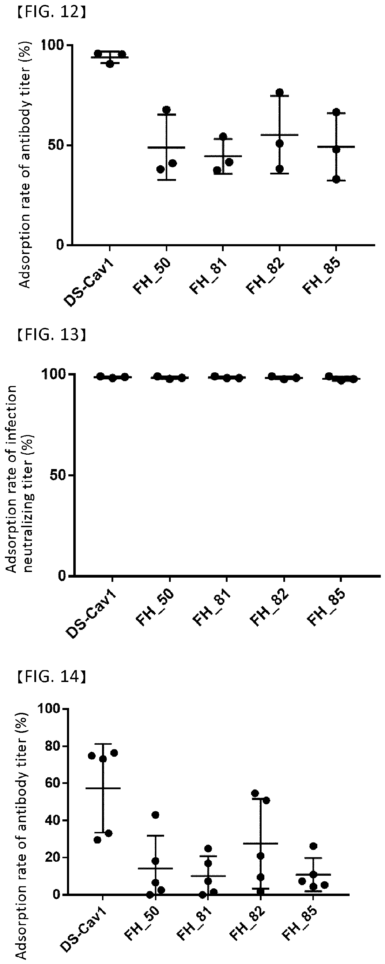

An ELISA test in which the antigen ΔFP furin_{wt} F_{ecto} was immobilized was carried out in order to confirm a degree of binding of an antibody (an antibody that is thought to have a relatively low viral infection neutralization capacity) that binds to the Post-F. A carbonate bicarbonate buffer (Sigma-Aldrich, C3041-50CAP) was used as an antigen-binding buffer, and TBS (TaKaRa, T9142) was used for diluting a sample and washing a plate. 2.0 pmol of the antigen ΔFP furin_{wt} F_{ecto} was added to a 96 half-well plate to be directly immobilized and blocking with bovine serum albumin was performed. A 4-fold dilution series was prepared by appropriately adding a buffer to a healthy adult serum or a healthy adult serum after an antigen adsorption operation, and was added to a plate and incubated (room temperature, 1 hour, stationary). After that, the diluted serum was removed, and a peroxidase-binding anti-human IgG antibody (Jackson Immuno Research, 709-035-149) was bound, and further, a color was developed with a standard peroxidase detection reagent, and then, an intensity of the color development was determined by an absorbance measurement using a standard plate reader. A relationship between a serum dilution rate and an absorbance was obtained by piecewise linear interpolation between measurement points, an inverse number of the dilution rate at which the absorbance is 0.2 was defined as an antibody titer of an antibody that binds to the post-F, and a relative value of a decrease in serum antibody titer after an adsorption operation when the serum antibody titer before the adsorption operation was 100% was defined as an adsorption rate (Fig. 12).

In order to confirm to what extent an antibody having a viral infection neutralization capacity was removed by an adsorption operation, infection evaluation was performed using an RS virus (ATCC, VR-1540) and a Vero cell (ATCC, CCL-81). A 5-fold dilution series was prepared by appropriately adding PBS (pH 7.4) to a healthy adult serum or a healthy adult serum after an antigen adsorption operation, and an RS virus was mixed and incubated (room temperature, 30 minutes, stationary), and then, an amount of 4×104 cells was seeded in each well of a 96-well plate, added to Vero cells cultured for 1 day, and incubated (37 °C, humidity, 2 hours, stationary). After that, a virus solution was removed, and then, a culture medium, in which a Sodium Pyruvate Solution (Nakarai, 06977-34) and an Antibiotic-Antimycotic Mixed Stock Solution (Nakarai, 09366-44) were added in an amount of 1% (v/v) each to DMEM and High Glucose (Thermo Fisher Scientific, 11965), was added in an amount of 100 µL per well, and was further incubated (37 °C, humidity, 16 hours, stationary). Next, cells were immobilized with paraformaldehyde, and, for cells permeabilized with Triton X-100 (Nakarai, 35501-15), nuclei of infected cells and all cells were stained using Anti-RSV Antibodies, fusion proteins, all type A, B strains, clone 133-1H, Alexa Fluor 488 (Merck, MAB8262X) and Heochst33258 (Dojindo, 343-07961), and the number of nuclei in an infected cell region was counted using an imager IN Cell Analyzer (GE Healthcare) to obtain the number of virus-infected cells. A relationship between a dilution rate x of each serum and the number of infections y was determined by fitting a sigmoid curve (y = Ymax/(1+ (x/ IC50) C)) to experimental results (fitting by a least-squares error method), and an inverse number of IC50 was used as a virus infection neutralizing titer. When a virus infection neutralizing titer of a serum before an adsorption operation was set to 100%, a relative value of a decrease in the virus infection neutralizing titer after the adsorption operation was taken as an adsorption rate (Fig. 13).

By the above operation, it was shown that, as compared to the antigen F_DS-Cav1 in the prior art document, the antigens FH_50, FH_81, FH_82, and FH_85 have a low binding property to a post-F recognition antibody present in a healthy adult serum, and only about half of antibodies can be bound in an adsorption operation. Nevertheless, it was also shown that almost all antibodies that contribute to a viral infection neutralization capacity are bound in the same manner as F_DS-Cav1, and antibodies remaining after an adsorption operation have almost no viral infection neutralization capacity. The above results suggest that, as compared to the introduction of the DS-Cavl mutation, the newly found mutation has less antibody binding that is ineffective in virus infection neutralization and, when used as a vaccine antigen, has a low risk of inducing an antibody that does not contribute to virus infection protection.

### (Verification of antibody induced by mouse immunity)

It has been suggested that, by the above operation, a mutant protein has an effect of suppressing induction of immunity that does not contribute to virus neutralization. In order to examine the effect in more detail, an analysis of antibodies induced by immunizing mice with the antigen FH_50, FH_81, FH_82, FH_85 or F_DS-Cav1 was performed.

Mouse immune serums were obtained by immunizing BALB/cAnNCrlCrlj (Japanese Charles River) (female, 7 weeks old) with the mutant proteins. A solution obtained by mixing well the antigen FH_50, FH_81, FH_82, FH_85 or F_DS-Cav1 (antigen concentration: 0.80 mg/mL), which was dissolved in PBS (pH 7.4), and aluminum hydroxide (InvivoGen, vac-alu-250) in equal amounts was intramuscularly administered in an amount of 50 µL to hind limb thighs of mice on the 0th day of breeding and on the 21st day of breeding (5 mice per group). Blood was obtained from a posterior vena cava on the 42nd day of breeding, and the obtained blood was incubated (room temperature, about 2 hours, stationary), and then, a serum was collected by centrifugation, and was inactivated (55 °C, 30 min) to prepare a mouse immune serum.

In order to confirm a ratio at which antibodies that bind to conformations after fusion were induced, each mouse immune serum was subjected to an antigen adsorption operation using magnetic beads Dynabeads His-Tag Isolation and Pulldown (Thermo Fisher Scientific, 10103D). First, 20 µg of ΔFP furin_{wt} F_{ecto} was applied to and caused to be bonded to beads of 45 µL of a bead solution washed twice with PBS (pH 7.4), and then, blocking with bovine serum albumin was performed. Next, 50 µL of each mouse immune serum was diluted 20-fold with PBS (pH 7.4), the beads were added thereto, and the mixture was incubated (4 °C, 1 hour, rotated), and then, removal of antibodies bound to antigens was performed by removing the beads.

Next, an ELISA test in which the antigen F_DS-Cav1 was immobilized was carried out, and a reduction rate of an anti-F antigen antibody by the antigen adsorption operation was measured. A carbonate bicarbonate buffer (Sigma-Aldrich, C3041-50CAP) was used as an antigen-binding buffer, and PBS (TaKaRa, T9183) was used for diluting a sample and washing a plate. 2.0 pmol of the antigen F_DS-Cav1 was added to a 96 half-well plate to be directly immobilized and blocking with bovine serum albumin was performed. A 5-fold dilution series was prepared by appropriately adding a buffer to a mouse immune serum or a mouse immune serum after an antigen adsorption operation, and was added to a plate and incubated (room temperature, 2 hours). After that, the diluted serum was removed, and a peroxidase-binding anti-mouse antibody (DaKo, P0161) was bound, and further, a color was developed with a standard peroxidase detection reagent, and then, an intensity of the color development was determined by an absorbance measurement using a standard plate reader. A relationship between a serum dilution rate and an absorbance was obtained by piecewise linear interpolation between measurement points, an inverse number of the dilution rate at which the absorbance is 0.2 was defined as an antibody titer of a detected anti-F antigen antibody, and a relative value of a decrease in serum antibody titer after an adsorption operation when the serum antibody titer before the adsorption operation was 100% was defined as an adsorption rate (Fig. 14).

By the above operation, it was shown that, as compared to the antigen F_DS-Cav1 in the prior art document, the antigens FH_50, FH_81, FH_82, and FH_85 had a lower induction ratio of the post-F recognition antibody. That is, a result was obtained suggesting that, when the antigens FH_50, FH_81, FH_82, FH_85 are used as vaccine antigens, the risk of inducing an antibody that does not contribute to virus infection protection is low.

### [Example 6] Second comparison with predecessor

The F protein moieties of the antigens FH_50, FH_81, FH_82, and FH_85 carried out in Example 5 all contain mutations of L141C and L373C. The mutations are mutations introduced in order to introduce a disulfide bond in Example 1. However, in a prior patent (International Publication No. 2017/109629), introduction of a disulfide bond to a relatively close site (Mutant ID: pXCS524.L142C, and N371C) is examined, and optimized antigens with further mutations (Mutant ID: pXCS881.L142C, N371C, S55C, L188C, T54H, V296I, D486S, E487Q, and D489S) are examined. Therefore, the useful antigens found in the above-described examples were compared with the antigen Pf524 or Pf881 into which the pXCS524 mutation or the pXCS881 mutation was introduced. Mutation contents of precursor proteins of antigens prepared below are summarized in Table 6.

### [Table 6]

**Table 6**

| Antigen name | Mutation content |
|---|---|
| Pf524 | L142C, N371C |
| FH_08 | L141C,L373C |
| Pf881 | L142C, N317C, S55C, L188C, T54H, V296I, D486S, E487Q, D489S |
| FH_82 | L141C, L373C, R135N, R136N, T189V, S190V, K42R, V384T |
| FH_85 | L141C, L373C, R135N, R136N, T189V, S190V, E60M, K42R, V384T |

As the antigens FH_08, FH_82, and FH85, those respectively described in Example 1, Example 3, and Example 5 were used. Polynucleotides encoding precursor proteins of Pf524 and Pf881 were each inserted into a pcDNA3.4 vector (Thermo Fisher Scientific, Inc.) using a standard genetic engineering technique, that a target DNA sequence has been inserted was confirmed by base sequence analysis, and mammalian cell expression vectors expressing the precursor proteins of the antigens were each constructed. A vector for introduction into a cell was purified using a standard plasmid vector purification method.

Expression and purification of each the antigens were carried out by the same operation as in Example 2.

An expression level of each antigen was measured by the same operation as in Example 3.

### (Antibody binding property test)

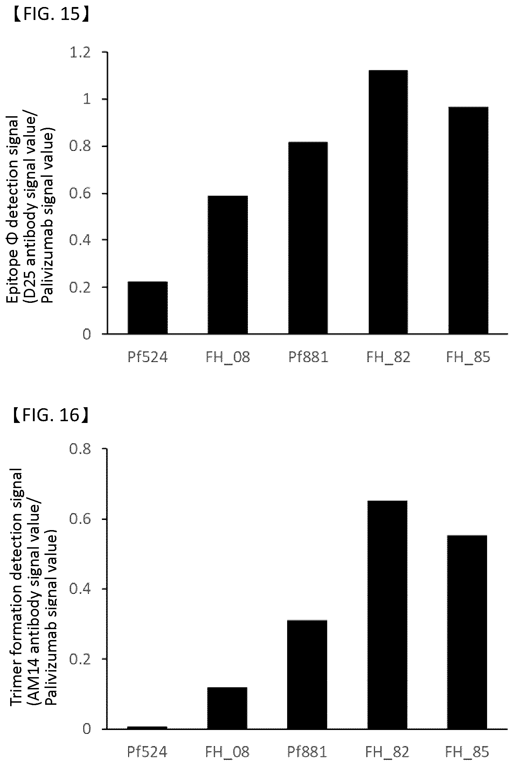

A binding property of each antigen to each antibody was measured by the same operation as in Example 5. However, a value of a binding signal was set to a value obtained by subtracting a detected value when PBS (pH 7.4) was applied for 180 seconds from a detected value when each antigen solution was applied for 180 seconds (Figs. 15 - 17).

By the above operation, a result was obtained showing that, for the antigen FH_08, as compared to the antigen Pf524 to which the mutation of the prior patent has been introduced, while the storage stability of the epitope Φ and the trimer formation are excellent, the epitope I, which is known to induce an antibody having a significantly low viral infection neutralization capacity, is hardly detected. Further, a result was obtained showing that, for the optimized antigens FH 82 and FH_85, similarly, as compared to the antigen Pf881 to which the mutation of the prior patent has been introduced, while the storage stability of the epitope Φ and the trimer formation are excellent, the epitope I, which is known to induce an antibody having a significantly low viral infection neutralization capacity, is hardly detected. This suggests that the useful disulfide introduction mutations found in the above example are mutations that preferentially induce a group of antibodies that are thought to have a higher effect on virus neutralization as compared to the disulfide introduction mutations (L142C and N371C) in the prior patent.

### (Verification of antibody induced by mouse immunity)

From the above experiment, it is suggested that the antigens FH 82 and FH_85 have an effect that induction of immunity that does not contribute to virus neutralization is lower as compared to that of the mutant Pf881 into which the mutation of the prior patent has been introduced. In order to examine the effect in more detail, an analysis of antibodies induced by immunizing mice with the antigen FH 82, FH_85 or Pf881 was carried out.

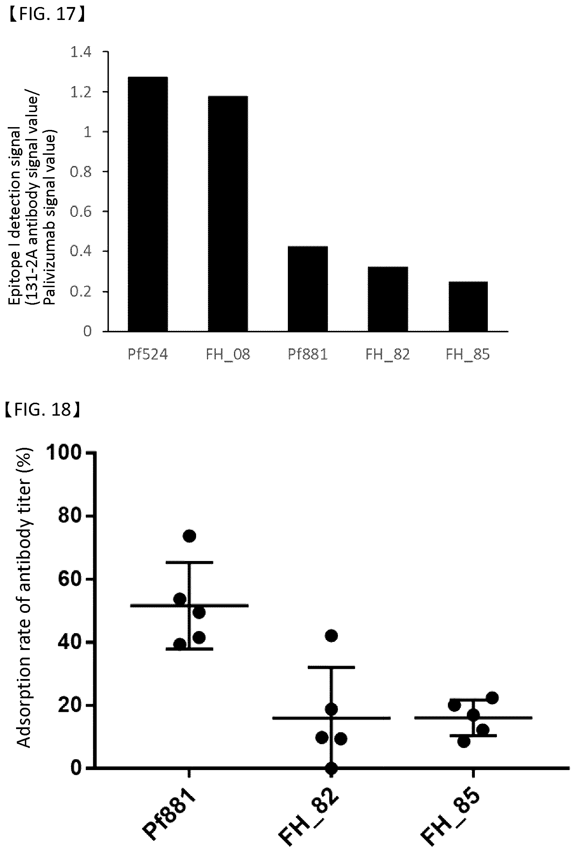

The analysis of the introduced antibodies was carried out by the same operation as in Example 5, and an adsorption rate of an antibody titer by an adsorption operation by ΔFP furin_{wt} F_{ecto} was measured (Fig. 18).

By the above operation, it was shown that, as compared to the antigen Pf881 into which the mutation of the prior patent has been introduced, the antigens FH 82 and FH_85 had a lower induction ratio of the post-F recognition antibody. That is, it is a result suggesting that, when the antigens FH 82 and FH_85 are used as vaccine antigens, a group of antibodies that are thought to have a higher effect on virus neutralization as compared to Pf881 are preferentially induced.

### [Example 7] Particle formation of antigen using scaffolding particles

In order to further reduce a risk of inducing antibodies that do not contribute to virus infection protection, it is thought to be effective to reduce, by a steric hindrance effect, the accessibility of antibodies or various immune cells to an antigen site recognized by the post-F recognition antibody such as the epitope I recognition antibody. Therefore, a possibility that the steric hindrance effect can be obtained by immobilizing antigens on appropriate scaffolding particles so that a recognition site of the post-F recognition antibody is on an inner side was considered.

However, when antigens are presented on suitable scaffolding particles, when a density is low because spacings between the antigens are equal to or longer a certain length, or when flexible linkers having a certain length or longer are introduced between the antigens and the scaffolding particles, it is thought there is a possibility that the above-described steric hindrance effect cannot be obtained. Therefore, GS linkers of multiple lengths were examined using HBsAg VLP which is thought to be able to achieve a relatively high-density antigen presentation.

### (Preparation of particulate antigen)

As scaffolding particles, Bionanocapsule-ZZ (Beacle Inc., BCL-DC-002) capable of binding an Fc fusion protein was used.

As precursor proteins of antigens, a precursor protein (SEQ ID NO: 31) of an antigen F DS-Cav1-Fc, a precursor protein (SEQ ID NO: 32) of FH_82-Fc, and a precursor protein (SEQ ID NO: 33) of FH_85-Fc, in each of which a sequence (SEQ ID NO: 22) containing a Thrombin recognition sequence, a His tag, a Strep-Tag II in the precursor proteins of F_DS-Cav1, FH 82, and FH_85 described above was substituted with an Fc sequence (SEQ ID NO: 30) were prepared. Further, regarding the precursor protein of the antigen F_DS-Cavl-Fc and the precursor protein of FH_85-Fc, those in which a GS linker (GGGGSGGGGSGGGGSGGGGS (SEQ ID NO: 75), GGGGSGGGGS (SEQ ID NO: 19) or GGGGS (SEQ ID NO: 27)) is inserted immediately before the Fc sequence were prepared based on the corresponding precursor proteins (SEQ ID NOs: 34 - 39) using the following method.

A polynucleotide encoding each of the above precursor proteins was inserted into a pcDNA3.4 vector (Thermo Fisher Scientific, Inc.) using a standard genetic engineering technique. That a target DNA sequence has been inserted was confirmed by a base sequence analysis, and mammalian cell expression vectors expressing the precursor proteins of the antigens were each constructed. A vector for introduction into a cell was purified using a standard plasmid vector purification method.

Expression of the precursor protein of each antigen was carried out by mammalian cell secretion expression using an Expi293 Expression System (Thermo Fisher Scientific, Inc.). Each purified vector was introduced into an Expi293 cell using an Expi Fectamin 293 Reagent, which is a gene transfer reagent of the same kit, each encoded antigen was secreted and expressed by culturing for about 4-5 days, and only a supernatant component was obtained by centrifugation and using a filter with a hole diameter of 0.22 µm.

Purification of each antigen was carried out using a standard affinity purification method using a Protein A purification column. After the purification, solvent substitution with PBS (pH 7.4) was performed using an ultrafiltration membrane.

Each of the prepared antigens was mixed with HBsAg VLP at a ratio of about 135 molecules (about 45 molecules as a trimer) per one HBsAg VLP particle on average, and was immobilized to scaffolding particles by allowing the mixture to stand at a room temperature for one hour. In this case, that there was substantially no unimmobilized antigen was confirmed by performing Blue Native PAGE.

The antigens used in the evaluation in the present example, and particulate antigens formed by immobilizing the antigens on the scaffolding particles (hereinafter, which may also be referred to as "particulate antigens") are summarized in Table 7.

**[Table 7]**

| Antigen name | Description |
|---|---|
| F_DS-Cavl | Prepared in Example 5 |
| F---------_DS-Cav1-Fc - | A sequence containing a Thrombin recognition sequence, a His tag, a Strep Tag II of F DS-Cavl is substituted with the Fc sequence |
| F_DS-Cav1-4GS-Fc - | GGGGSGGGGSGGGGSGGGGS (SEQ ID NO: 75) is inserted immediately before the Fc sequence of F DS-Cav1-Fc |
| F_DS-Cav1-2GS-Fc F_DS-Cav1-2GS-Fc | GGGGSGGGGS (SEQ ID NO: 19) is inserted immediately before the Fc sequence of F_DS-Cav1-Fc |
| F_DS-Cav1-1GS-Fc - | GGGGS (SEQ ID NO: 27) is inserted immediately before the Fc sequence of F DS-Cav1-Fc |
| F DS-Cav1-VLP | F DS-Cav1-Fc is immobilized on HBsAg VLP |
| F_DS-Cav1-4GS- VLP | F_DS-Cav1-4GS-Fc is immobilized on HBsAg VLP |
| F_DS-Cav1-2GS- VLP | F_DS-Cav1-2GS-Fc is immobilized on HBsAg VLP |
| F_DS-Cav1-1GS- VLP | F_DS-Cavl-1GS-Fc is immobilized on HBsAg VLP |
| FH 82 | Prepared in Example 5 |
| FH_82-Fc | A sequence containing a Thrombin recognition sequence, a His tag, a Strep Tag II of FH 82 is substituted with the Fc sequence |
| FH 82-VLP | FH 82-Fc is immobilized on HBsAg VLP |
| FH 85 | Prepared in Example 5 |
| FH_85-Fc | A sequence containing a Thrombin recognition sequence, a His tag, a Strep Tag II of FH 85 is substituted with the Fc sequence |
| FH_85-4GS-Fc | GGGGSGGGGSGGGGSGGGGS (SEQ ID NO: 75) is inserted immediately before the Fc sequence of FH_85-Fc |
| FH_85-2GS-Fc | GGGGSGGGGS (SEQ ID NO: 19) is inserted immediately before the Fc sequence of FH_85-Fc |
| FH_85-1GS-Fc | GGGGS (SEQ ID NO: 27) is inserted immediately before the Fc sequence of FH_85-Fc |
| FH_85-VLP | FH_85-Fc is immobilized on HBsAg VLP |
| FH_85-4GS-VLP | FH_85-4GS-Fc is immobilized on HBsAg VLP |
| FH_85-2GS-VLP | FH_85-2GS-Fc is immobilized on HBsAg VLP |
| FH_85-1GS-VLP | FH_85-1GS-Fc is immobilized on HBsAg VLP |

### (Antibody binding property test)

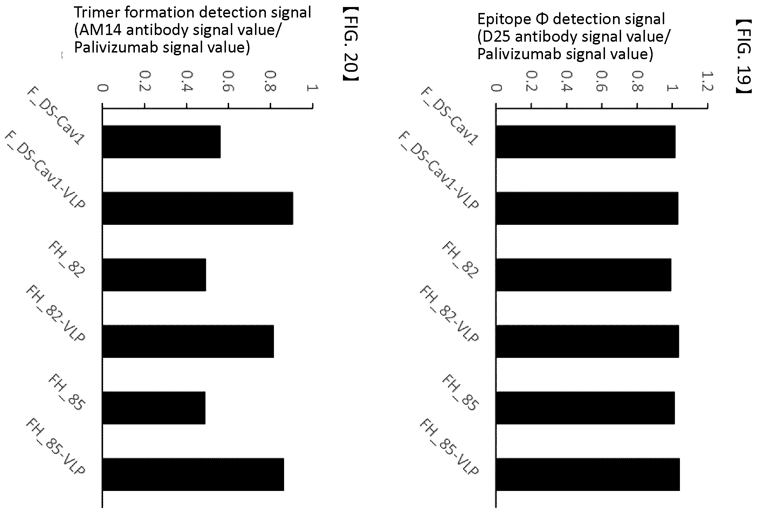

Binding properties of F_DS-Cav1, F_DS-Cav1-VLP, FH 82, FH 82-VLP, FH_85 and FH_85-VLP, which are antigens or particulate antigens, to the antibodies were measured by the same operation as in Example 6 (Figs. 19 - 21).

By the above operation, it was shown that, regardless of a pre-stabilization method of an F antigen region (content of an amino acid mutation for pre-stabilization), when the pre-F is immobilized on scaffolding particles via a domain added to a C-terminal of an amino acid sequence thereof, trimer formation is improved while a binding property of an antibody with respect to the epitope Φ is maintained. Further, it was shown that a detection value of the epitope I, which is known to induce an antibody that has a significantly low viral infection neutralization capacity, is reduced. The above result suggests that when the particulate antigens are used as vaccine antigens, a group of antibodies that are thought to have a high effect on virus neutralization are preferentially induced.

### (Examination of insertion linker)

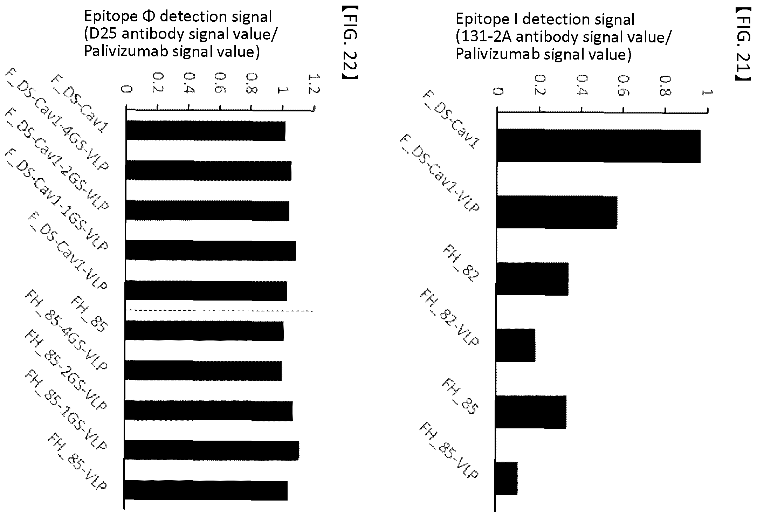

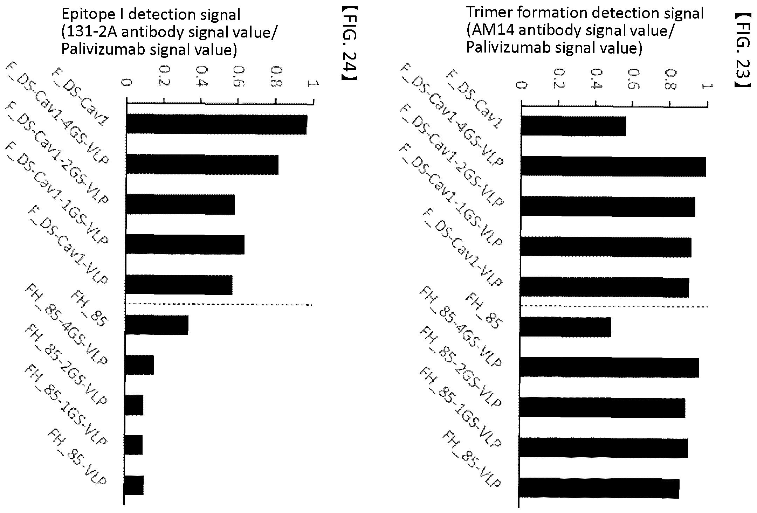

Binding properties of F_DS-Cav1, F_DS-Cav1-4GS-VLP, F_DS-Cav1-2GS-VLP, F_DS-Cav1-1GS-VLP, F_DS-Cav1-VLP, FH 85, FH 85-4GS-VLP, FH 85-2GS-VLP, FH 85-1GS-VLP and FH_85-VLP, which are antigens or particulate antigens, to the antibodies were measured by the same operation as in Example 6 (Figs. 22 - 24).

By the above operation, it was shown that, by the carried out insertion of any of the GS linkers, an effect is achieved that trimer formation is improved while a binding property of an antibody with respect to the epitope Φ is maintained, and the detection value of the epitope I is reduced. However, it was shown that the effect of reducing the detection of the epitope I is decreased when a GS linker that is too long is inserted between the scaffolding particles and the antigen. Specifically, a significant influence was not seen for a length of about GGGGSGGGGS (SEQ ID NO: 19). However, for a longer length of GGGGSGGGGSGGGGSGGGGS (SEQ ID NO: 75), the decrease in the effect of reducing the detection of the epitope I was seen.

The above result suggests that, when a flexible linker having a certain length or longer is inserted between the scaffolding particles and the antigen, the effect of reducing the accessibility of the post-F recognition antibody to a recognition site by the steric hindrance effect is decreased.

### (Estimation of antigen density)

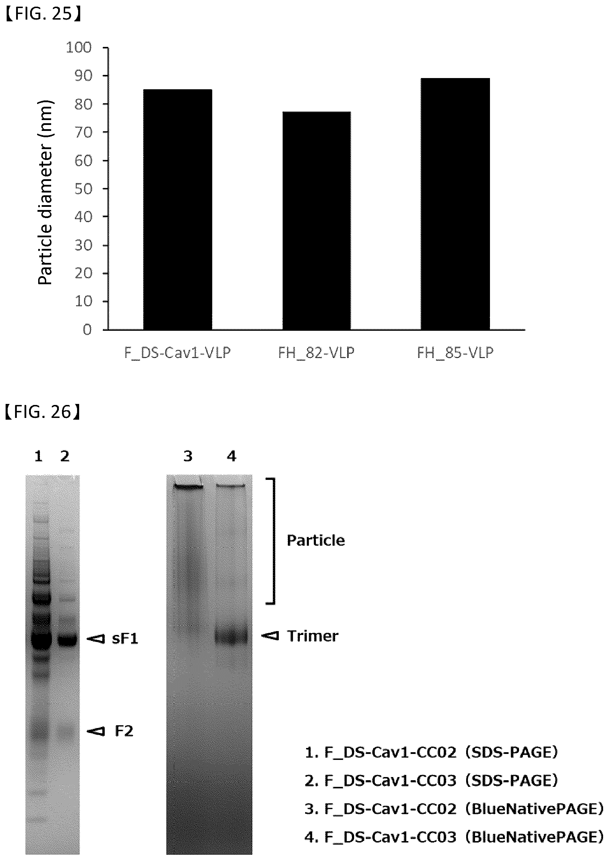

A theoretical diameter of the particulate antigen FH_85-VLP was estimated using a dynamic light scattering method using Zetasizer µV (Malvern), and was found to be 89.28 nm (Fig. 25). Since about 135 molecules (about 45 molecules as a trimer) per one particle on average are presented, an average molecular density per 1 nm² of a theoretical surface area can be calculated to be about 0.005 molecules (about 0.0018 molecules as a trimer). Here, a surface area (S) of a sphere was calculated according to the following formula using a radius (r) thereof. $S = 4 {πr}^{2}$

That is, it is suggested that the desired steric hindrance effect can be achieved when the density is such that at least about 0.005 antigen molecules or more are present per 1 nm² of the theoretical surface area estimated using the dynamic light scattering method.

The same result was also obtained for DS-Cav1-VLP and FH_82-VLP (Fig. 25).

### (Verification of antibody induced by mouse immunity)

From the above experiment, it is suggested that the pre-F antigen bound to the scaffolding particles HBsAg VLP has an effect of suppressing the induction of immunity that does not contribute to virus neutralization. In order to examine the effect further in more detail, an analysis of antibodies induced by immunizing mice with F_DS-Cav1, F_DS-Cav1-VLP, FH_82, FH_82-VLP, FH_85 or FH_85-VLP is carried out.

Mouse immune serums are obtained by immunizing BALB/c (female, 5 - 8 weeks old) with the particulate antigens. As a solvent composition of each of the particulate antigens, a well-mixed solution of PBS or PBS and aluminum hydroxide (InvivoGen, vac-alu-250) in equal amounts is used. The antigen is intramuscularly administered to hind limb thighs of the mice twice at an interval of about 3 weeks, and blood is obtained about 3 weeks after the second administration. The obtained blood is incubated at a room temperature and then is centrifuged to collect a serum.

An ELISA test in which the antigen of each conformation is immobilized is carried out in order to confirm a degree of induction of an antibody that binds to the pre-F or the post-F in the serum.

Further, an antigen adsorption operation and a measurement of a reduction rate of an antigen recognition antibody due to the antigen adsorption operation are carried out in order to calculate an induction rate of the post-F recognition antibody. A method therefor conforms to the method shown in Example 5.

In conclusion, by the particle formation, the effect of preferentially inducing a group of antibodies that are thought to have a high effect on virus neutralization is confirmed.

### [Example 8] Particle formation of antigen using a hydrophobic peptide chain

A possibility was considered that the effect of reducing the accessibility of antibodies or various immune cells with respect to an antigen site recognized by the post-F recognition antibody shown in Example 7 by the steric hindrance effect can also be realized using a method that does not use scaffolding particles. Therefore, in the present example, particle formation by hydrophobic aggregation using a hydrophobic peptide chain (a polypeptide chain containing a hydrophobic amino acid) was examined.

### (Examination of hydrophobic peptide chain)

A hydrophobic peptide chain to be used needs to have a property of hydrophobically aggregating under a vaccine preparation or in vivo condition, but is difficult to be put into practical use for a sequence that significantly reduces efficiency of secretion expression of an antigen due to cells. In general, it is known that, when a polypeptide chain containing a large amount of hydrophobic amino acids is fused to a protein, production efficiency of the protein in cells decreases. Therefore, suitable hydrophobic polypeptide chains were examined.

Antigens were prepared based on precursor proteins in which a sequence (SEQ ID NO: 22) containing a Thrombin recognition sequence, a His tag, and a Strep-Tag II in a precursor protein of the antigen F_DS-Cav1 is substituted with sequences (SEQ ID NOs: 44 - 47) that contain various hydrophobic peptide chains (SEQ ID NOs: 40 - 43) and a FLAG tag which is a tag for purification. Examined hydrophobic peptide sequences are summerized in Table 8-1.

A polynucleotide encoding each of the above precursor proteins was inserted into a pcDNA3.4 vector (Thermo Fisher Scientific, Inc.) using a standard genetic engineering technique. That a target DNA sequence has been inserted was confirmed by a base sequence analysis, and mammalian cell expression vectors expressing the precursor proteins were each constructed. A vector for introduction into a cell was purified using a standard plasmid vector purification method.

Expression of each of the antigens was carried out by the same operation as in Example 7.

Purification of each antigen was carried out by affinity purification using ANTI-FLAG M2 Affinity Gel (Sigma, A2220). An FLAG peptide was used for elution of the affinity purification. However, the FLAG peptide in each antigen sample was removed using an ultrafiltration membrane. PBS (pH 7.4) was used as a solvent.

Feasibility of secretion expression was determined by performing a general SDS-PAGE after purification and confirming whether or not a target protein could be separated and purified with high purity. Further, feasibility of particle formation was confirmed by performing a general Blue Native PAGE after purification.

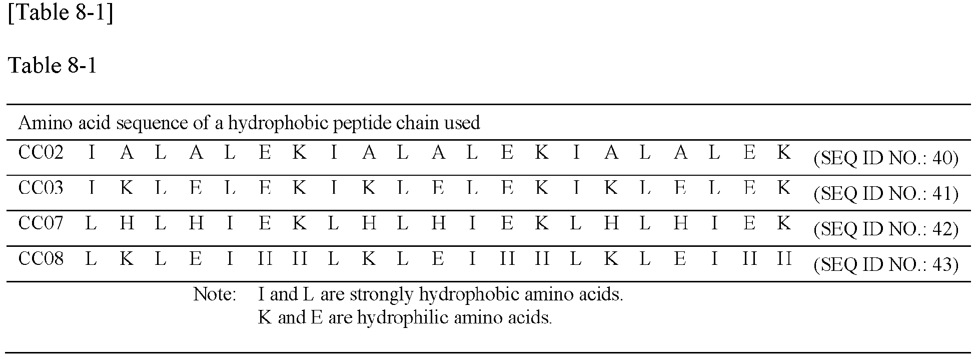

Feasibility of secretion expression and feasibility of particle formation of F_DS-Cav1-CC02 and F_DS-Cav1-CC03 were examined, and it was found that F_DS-Cav1-CC03 secretes and expresses but does not form particles, whereas F_DS-Cav1-CC02 does not secrete and express well (Fig. 26). Therefore, it was thought that an optimal hydrophobicity of a hydrophobic peptide chain to be used is between hydrophobicities of the two.

The difference between the hydrophobic peptide chains of F_DS-Cav1-CC02 and F_DS-Cav1-CC03 is that an alanine which is a non-hydrophilic amino acid is substituted with a lysine or a glutamic acid which is a hydrophilic amino acid. Therefore, first, it was considered to change the alanine of F_DS-Cav1-CC02 to a histidine which has a moderate hydrophilicity and a pK of about pH 6. Since it is known that the pH of a solvent during preparation of a pharmaceutical formulation is near neutral, whereas the pH in a secretory pathway of cells is lower, it was expected that a peptide chain containing a histidine has a relatively hydrophilic property during secretion expression, and a relatively hydrophobic property during preparation of a pharmaceutical formulation.

Further, assuming that a designed hydrophobic peptide chain forms a coiled-coil structure, it is expected that phases of 21 amino acids of a hydrophobic peptide chain connected directly under a foldon domain are defgabcdefgabcdefgabc in this order. When a leucine and an isoleucine are arranged at positions (a) and (d) that hydrophobically interact in a trimer, according to a previous study (Joel P. Schneider et al., 1998, Folding & Design, 3: R29) examining amino acid sequences of natural proteins, since an isoleucine at a position (a) and a leucine at a position (d) are more frequent, it was considered to replace a leucine and an isoleucine at a position (a) and a position (d) of F_DS-Cav1-CC02.

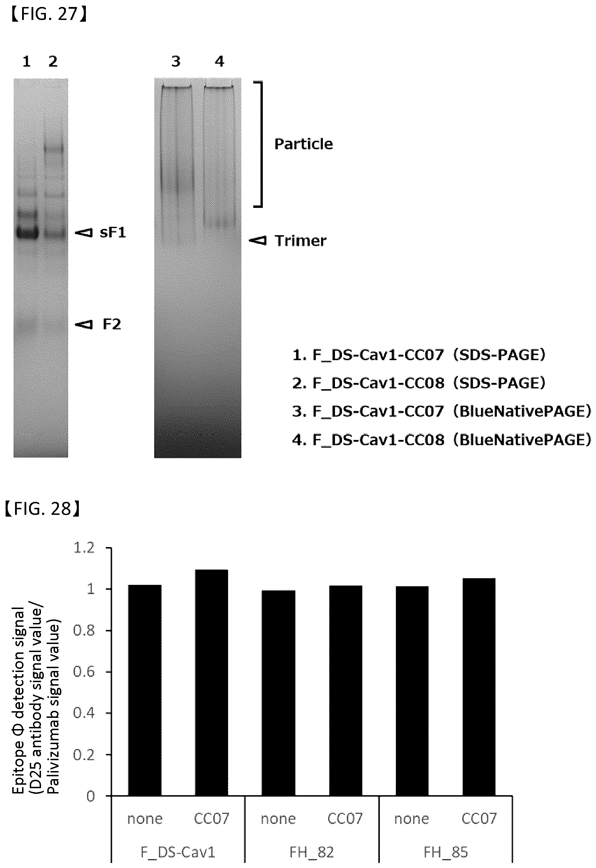

From the above, F_DS-Cav1-CC07 was newly designed and feasibility of secretion expression and feasibility of particle formation were examined, and both secretion expression and particle formation were achieved (Fig. 27). Since secretion expression is difficult for F_DS-Cav1-CC08 in which positions of a histidine, a lysine and a glutamic acid are changed, it is also suggested that it is not simply a problem only of a composition of an amino acid, a three-dimensional arrangement is important. 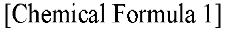 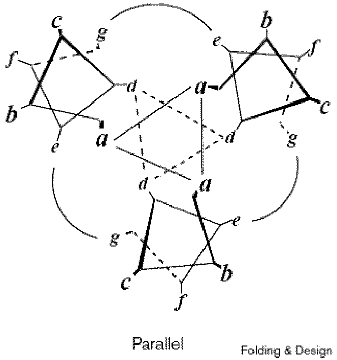 (quoted from Folding & Design 1998, 3:R29)

### (Preparation of particulate antigen)

Antigens were prepared based on precursor proteins (SEQ ID NOs: 48 and 49) in which a sequence (SEQ ID NO: 22) containing a Thrombin recognition sequence, a His tag, and a Strep Tag II in precursor proteins of the antigens FH_82 and FH_85 is substituted with a sequence that contains an optimal hydrophobic peptide chain (SEQ ID NO: 42) and a FLAG tag which is a tag for purification.

A polynucleotide encoding the above precursor proteins was inserted into a pcDNA3.4 vector (Thermo Fisher Scientific, Inc.) using a standard genetic engineering technique. That a target DNA sequence has been inserted was confirmed by a base sequence analysis, and mammalian cell expression vectors expressing the precursor proteins were each constructed. A vector for introduction into a cell was purified using a standard plasmid vector purification method.

Expression of each antigen was carried out by the same operation as in the examination of the hydrophobic peptide chains.

Purification of each antigen was carried out by the same operation as in the examination of the hydrophobic peptide chains.

Antigens (which may also be referred to as "particulate antigens") used in evaluation later in this example are summarized in Table 8-2.

### [Table 8-2]

**Table 8-2**

| Antigen name | Description |
|---|---|
| F DS-Cavl | Prepared in Example 5 |
| F_DS-Cav1-CC07 | A sequence containing a Thrombin recognition sequence, a His tag, a Strep Tag II of F_DS-Cav1 is substituted with a sequence containing a hydrophobic peptide chain "LHLHIEKLHLHIEKLHLHIEK" (SEQ ID NO: 42) and a FLAG tag |
| FH 82 | Prepared in Example 5 |
| FH_82-CC07 | A sequence containing a Thrombin recognition sequence, a His tag, a Strep Tag II of FH_82 is substituted with a sequence containing a hydrophobic peptide chain "LHLHIEKLHLHIEKLHLHIEK" (SEQ ID NO: 42) and a FLAG tag |
| FH_85 | Prepared in Example 5 |
| FH_85-CC07 | A sequence containing a Thrombin recognition sequence, a His tag, a Strep Tag II of FH_85 is substituted with a sequence containing a hydrophobic peptide chain "LHLHIEKLHLHIEKLHLHIEK" (SEQ ID NO: 42) and a FLAG tag |

### (Antibody binding property test)

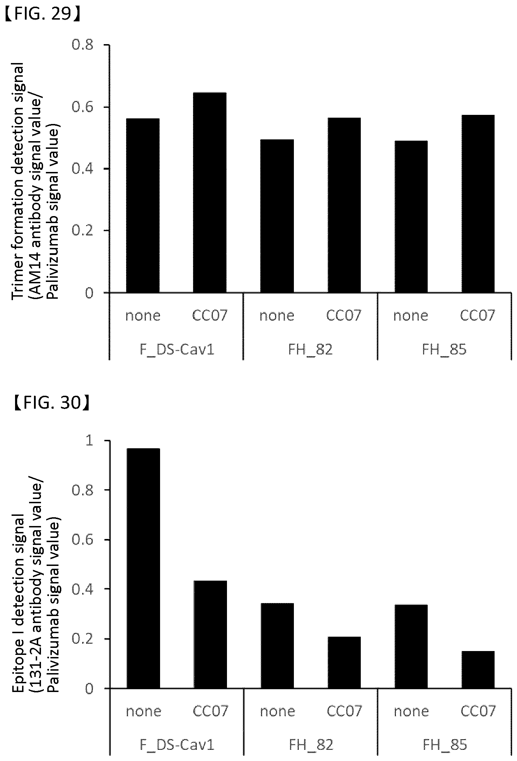

Binding properties of the prepared particulate antigens to antibodies were measured by the same operation as in Example 6 (Figs. 28 - 30).

By the above operation, a result was obtained showing that, regardless of a pre-stabilization method of an F antigen region (content of an amino acid mutation for pre-stabilization), a particulate antigen aggregated by a hydrophobic peptide chain having a pre-F added to a C-terminal of an amino acid sequence thereof improves trimer formation while maintaining a binding property of an antibody with respect to the epitope Φ. Further, a result was obtained showing that the detection of the epitope I, which is known to induce an antibody that has significantly low viral infection neutralization capacity, is reduced. The above results suggest that, when the antigen is used as a vaccine antigen, by the particle formation, the effect of preferentially inducing a group of antibodies that are thought to have a high effect on virus neutralization is improved.

### (Estimation of antigen density)

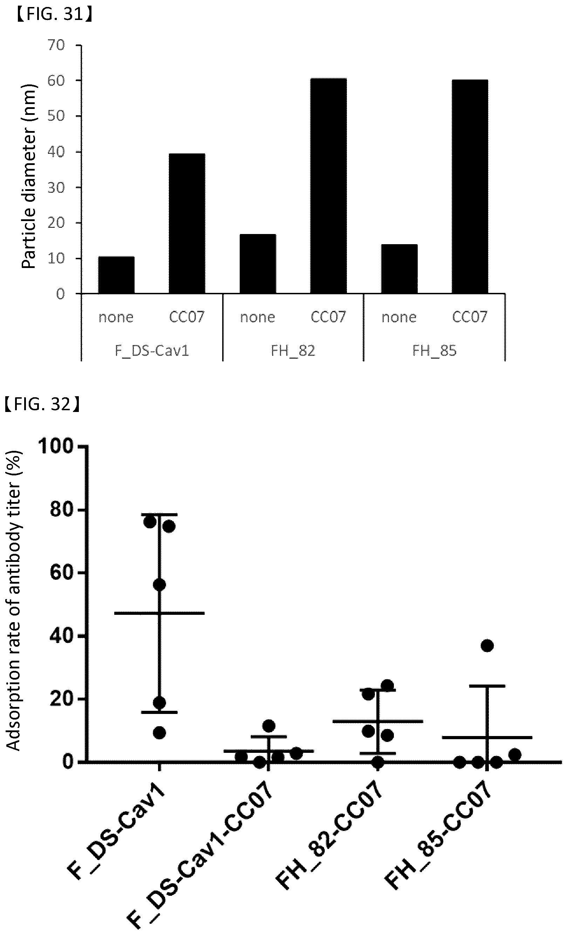

Theoretical diameters of the antigen FH_85 and the particulate antigen FH 85-CC07 were estimated using a dynamic light scattering method using DynaPro Plate Reader III (Wyatt), and were found to be 13.8 nm and 60.0 nm (Fig. 31). Since a ratio of molecular weights of two particles is proportional to a ratio of their theoretical diameters to the 2nd to 3rd power for general proteins, it is estimated that a molecular weight of FH 85-CC07 is about 20 - 80 times a molecular weight of FH_85. That is, FH85-CC07 is thought to present about 60 - 240 molecules per particle (about 20 - 80 molecules as a trimer), and an average molecular density per 1 nm² of a theoretical surface area can be calculated to be about 0.005 - 0.022 molecules (about 0.0017 - 0.0073 molecules as a trimer).

The same results were obtained for FH_82-CC07 (Fig. 31).

Therefore, it was estimated that the particulate antigen using a hydrophobic peptide chain has an antigen density equivalent to or higher than that of the particulate antigen using scaffolding particles shown in Example 6.

### (Verification of antibody induced by mouse immunity)

By the above operation, it is suggested that the particle formation of an antigen to which a hydrophobic peptide chain is bound has an effect of suppressing induction of immunity that does not contribute to virus neutralization. In order to examine the effect in more detail, an analysis of an antibody induced by immunizing a mouse with the antigen or the particulate antigen F_DS-Cav1F_DS-Cav1-CC07, FH 82-CC07 or FH_85-CC07 was carried out.

Mouse immune serums were obtained by immunizing BALB/cAnNCrlCrlj (Charles River Laboratories Japan) (female, 7 weeks old) with the antigens and the particulate antigens. The antigen or particulate antigen F_DS-Cav1, F_DS-Cav1-CC07, FH_82-CC07 or FH_85-CC07 (antigen concentration: 0.40 mg/mL) dissolved in PBS (pH 7.4) was intramuscularly administered in an amount of 50 µL to hind limb thighs of mice on the 0th day of breeding and on the 21st day of breeding (5 mice per group). Blood was obtained from a posterior vena cava on the 42nd day of breeding, and the obtained blood was incubated (room temperature, about 2 hours, stationary), and then, a serum was collected by centrifugation, and was inactivated (55 °C, 30 min) to prepare a mouse immune serum.

In order to confirm a ratio at which antibodies that bind to conformations after fusion were induced, using the same method as in Example 6, each of the mouse immune serums was subjected to an antigen adsorption operation using magnetic beads to which ΔFP furin_{wt} F_{ecto} was bound or magnetic beads that were only subjected to a blocking operation without binding any antigen. Further, for each of the serums after the adsorption operation, an ELISA test was carried out in which F_DS-Cav1 was immobilized using the same method as in Example 6, and an adsorption rate was calculated. However, the adsorption rate was set to a relative value of a value obtained by subtracting an antibody titer of a serum after an adsorption operation due to ΔFP furin_{wt} F_{ecto} from an antibody titer of a serum after an adsorption operation without an antigen when the antibody titer of the serum after the adsorption operation without an antigen was set to 100% (Fig. 32).

By the above operation, it was shown that the post-F recognition antibody, which was about half induced by the antigen F_DS-Cav1 before particle formation, was hardly induced by the antigen F_DS-Cav1-CC07 that formed particles by a hydrophobic peptide chain. Further, similarly, the post-F recognition antibody was also hardly induced by FH_82-CC07 and FH_85-CC07. From the above, it is suggested that, by the particle formation, the effect of preferentially inducing a group of antibodies that are thought to have a high effect on virus neutralization is improved.

### [Example 9] Particle formation of antigen using self-associating protein domain

A possibility was considered that the effect of reducing the accessibility of antibodies or various immune cells with respect to an antigen site recognized by the post-F recognition antibody shown in Examples 7 and 8 by the steric hindrance effect can also be realized by fusion of a self-associating protein domain to a C-terminal side of an antigen. In particular, by regularly arranging antigens using a self-associating protein domain, a possibility was considered of obtaining a higher steric hindrance effect than the particle formation by hydrophobic aggregation shown in Example 8.

Therefore, in this example, particle formation utilizing a self-associating property of a core antigen (HBcAg) of the hepatitis B virus was examined.

However, for those obtained by substituting a sequence (SEQ ID NO: 22) containing a Thrombin recognition sequence, a His tag, and a Strep-Tag II in the antigen F_DS-Cav1 with a peptide sequence (SEQ ID NO: 51) that contains a GS linker (GGGGSGGGGSGGGGSGGGGS) (SEQ ID NO: 75), a HBcAg self-associating domain (SEQ ID NO: 50), and a His tag, although expression was observed, self-association was not observed (verification result by Blue Native PAGE). An N-terminal of the HBcAg self-associating domain exists near a binding surface when HBcAg self-associates, and a possibility was considered that self-association may be hindered by fusing a large protein to the N-terminal.

Therefore, in order to arrange an RSV F antigen moiety at a position far from the binding surface during self-association, when HBcAg self-associates, it is divided into a C-terminal side and an N-terminal side rather than an outwardly protruding site of a particle (a moiety between helices existing in an α3 domain and an α4 domain). Further, in order to reduce a risk of unexpected disulfide bond formation, two point mutations (C48S and C107A) that are thought to have no influence on self-association were introduced. Specifically, antigens (which may also be referred to as "particulate antigens") were prepared based on a precursor protein (SEQ ID NO: 55) of F_DS-Cav1-HBcCN and a precursor protein (SEQ ID NO: 56) of FH_85-HBcCN, in each of which a sequence (SEQ ID NO: 22) containing a Thrombin recognition sequence, a His tag and a Strep-Tag II in precursor proteins of the antigens F_DS-Cav1 and FH 85 was substituted with a GS linker (GGGGSGGGGSGGGGSGGGGS) (SEQ ID NO: 75), a C-terminal side of a HBcAg self-associating domain (SEQ ID NO: 52), and a sequence (SEQ ID NO: 54) in which a GS linker (GGGGSGGGGSGGGGSGGGGS) (SEQ ID NO: 20), an N-terminal side of a HBcAg self-associating domain (SEQ ID NO: 53) and an FLAG tag are bound in this order.

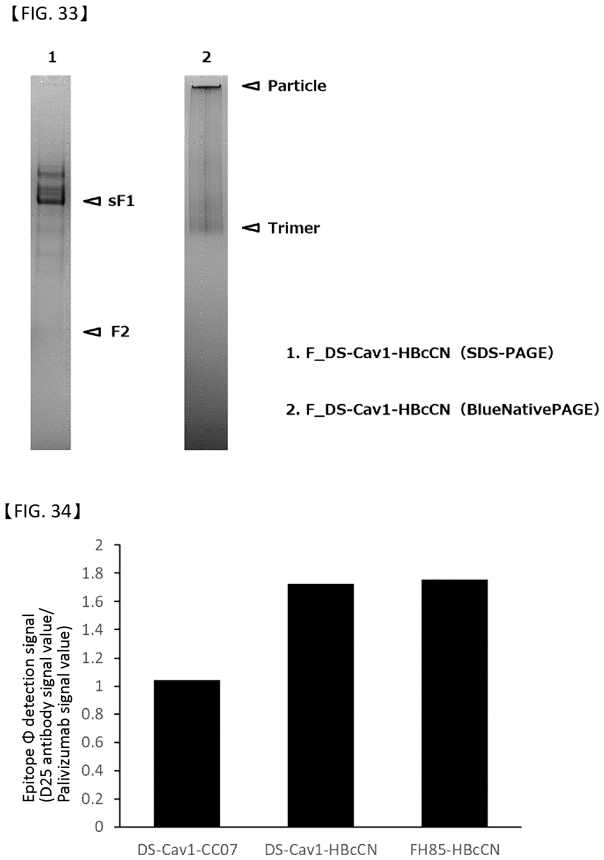

F_DS-Cav1-HBcCN was expressed and purified by the same operation as in Example 8 and feasibility of secretion expression and feasibility of particle formation were examined, and both secretion expression and particle formation were achieved (Fig. 33). However, since a relative expression level was low, improvement in the expression method was desired. Therefore, for a polynucleotide encoding a precursor of DS-Cav1-HBcCN or FH_85-HBcCN, a stable expression CHO line was established using a GS Xceed Gene Expression System (Lonza). The established cell line was cultured, and after each antigen was secreted and expressed, a culture supernatant component was obtained.

Purification of each particulate antigen was carried out by the same operation as in Example 8.

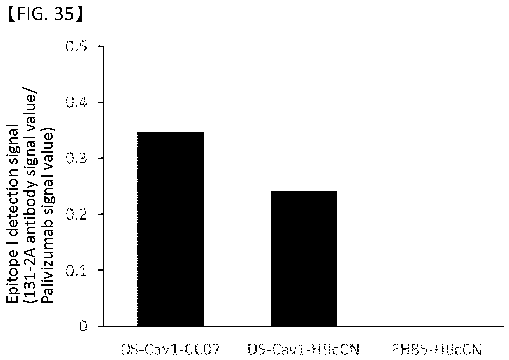

In order to confirm a binding property of each particulate antigen to each antibody, an ELISA test was carried out in which Palivizumab (a Synagis intramuscular injection, AbbVie, Inc.), a 131-2A antibody, and a D25 antibody were each immobilized. A carbonate bicarbonate buffer (Sigma-Aldrich, C3041-50CAP) was used as an antibody-binding buffer, and TBS (TaKaRa, T9142) was used for diluting a sample and washing a plate. 10 µg/mL of the Palivizumab or the 131-2A antibody or the D25 antibody was added to a 96 half-well plate to be directly immobilized and to be blocked with bovine serum albumin. A 10-fold dilution series was prepared by appropriately adding a buffer to each antigen, and was added to a plate to be incubated (room temperature, 2 hours, stationary). After that, the added antigen solution was removed, and a peroxidase-binding anti-FLAG M2 antibody (Sigma-Aldrich, A8592) was bound, and further, a color was developed with a standard peroxidase detection reagent, and then, an intensity of the color development was determined by an absorbance measurement using a standard plate reader. A relationship between the dilution rate of the antigen and the absorbance was determined by piecewise linear interpolation between measurement points, and a reciprocal of the dilution rate at which the absorbance was 0.2 was used as a binding force of the added antigen with respect to the immobilized antibody. A value obtained by dividing the binding force with respect to the D25 antibody by the binding force with respect to the Palivizumab was defined as an epitope Φ detection signal, and a value obtained by dividing the binding force with respect to the 131-2A antibody by the binding force with respect to the Palivizumab was defined as an epitope I detection signal (Figs. 34 and 35).

By the above operation, a result was obtained showing that, as compared to the particle formation by a hydrophobic peptide chain, a particulate antigen aggregated by a self-associating protein domain with a pre-F added to a C-terminal of an amino acid sequence thereof further improves a binding property of an antibody with respect to the epitope Φ, and reduces the detection of the epitope I which is known to induce an antibody having a significantly low viral infection neutralization capacity. The above result suggests that, by the particle formation, the effect of preferentially inducing a group of antibodies that are thought to have a high effect on

## Claims

1. A mutant RSV F protein having a mutation, wherein the mutation is a substitution of a leucine corresponding to a leucine at position 141 of an amino acid sequence of SEQ ID NO: 1 or a leucine corresponding to a leucine at position 142 with a cysteine, and a substitution of a leucine corresponding to a leucine at position 373 with a cysteine, and a disulfide bond is formed between the cysteines.

2. The mutant RSV F protein according to claim 1, wherein the mutant RSV F protein is derived from an RSV subtype A or an RSV subtype B.

3. The mutant RSV F protein according to claim 2, wherein the RSV subtype A is an RSV A2 strain or an RSV Long strain.

4. The mutant RSV F protein according to claim 2, wherein the RSV subtype B is an RSV 18537 strain.

5. The mutant RSV F protein according to any one of claims 1 to 4, wherein the mutant RSV F protein comprises an amino acid sequence having an identity of 85% or more to SEQ ID NO: 2 wherein a leucine corresponding to a leucine at position 141 of the amino acid sequence of SEQ ID NO: 1 or a leucine corresponding to a leucine at position 142 of the amino acid sequence of SEQ ID NO: 1, and a leucine corresponding to a leucine at position 373 of the amino acid sequence of SEQ ID NO: 1 are substituted with cysteines, and a disulfide bond is formed between the cysteines, thereby having an ability to induce an antibody against an RSV pre-type F protein.

6. The mutant RSV F protein according to any one of claims 1 to 4, wherein an amino acid that forms a furin recognition site that exists on a C-terminal side of a pep27 region is substituted such that the furin recognition site is not recognized by furin.

7. The mutant RSV F protein according to claim 6, wherein an amino acid that forms the furin recognition site is substituted with a non-basic amino acid, and the amino acid that forms the furin recognition site is an amino acid selected from the group consisting of an arginine corresponding to an arginine at position 133 of the amino acid sequence of SEQ ID NO: 1, an arginine corresponding to an arginine at position 135 of the amino acid sequence of SEQ ID NO: 1, and an arginine corresponding to an arginine at position 136 of the amino acid sequence of SEQ ID NO: 1.

8. The mutant RSV F protein according to claim 6 or 7, wherein the mutant RSV F protein comprises an amino acid sequence having an identity of 85% or more to SEQ ID NO: 3 wherein a leucine corresponding to a leucine at position 141 of the amino acid sequence of SEQ ID NO: 1 or a leucine corresponding to a leucine at position 142 of the amino acid sequence of SEQ ID NO: 1, and a leucine corresponding to a leucine at position 373 of the amino acid sequence of SEQ ID NO: 1 are substituted with cysteines, and further, an amino acid selected from a group consisting of an arginine corresponding to an arginine at position 133 of the amino acid sequence of SEQ ID NO: 1, an arginine corresponding to an arginine at position 135 of the amino acid sequence of SEQ ID NO: 1, and an arginine corresponding to an arginine at position 136 of the amino acid sequence of SEQ ID NO: 1 is substituted with a non-basic amino acid, and a disulfide bond is formed between the cysteines, thereby having an ability to induce an antibody against an RSV pre-type F protein.

9. The mutant RSV F protein according to claim 7 or 8, wherein the non-basic amino acid is an asparagine.

10. The mutant RSV F protein according to any one of claims 1 to 9, wherein a threonine corresponding to a threonine at position 189 of the amino acid sequence of SEQ ID NO: 1 and/or a serine corresponding to a serine at position 190 of the amino acid sequence of SEQ ID NO: 1 are substituted with hydrophobic amino acids.

11. The mutant RSV F protein according to claim 10, wherein the hydrophobic amino acids are each independently selected from a group consisting of a valine, an isoleucine, and a leucine.

12. The mutant RSV F protein according to any one of claims 1 to 11, wherein a lysine corresponding to a lysine at position 42 of the amino acid sequence of SEQ ID NO: 1 is substituted with an arginine and/or a valine corresponding to a valine at position 384 of the amino acid sequence of SEQ ID NO: 1 is substituted with a threonine.

13. A mutant RSV F protein having a mutation, wherein the mutation is a substitution of a glutamic acid corresponding to a glutamic acid at position 60 of an amino acid sequence of SEQ ID NO: 1 with a non-acidic amino acid.

14. The mutant RSV F protein according to claim 13, wherein the non-acidic amino acid is selected from a group consisting of a methionine, a phenylalanine, a leucine, a threonine, and a serine.

15. A fusion protein comprising: the mutant RSV F protein according to any one of claims 1 to 14; and a multimerization domain fused to a C-terminal of the mutant RSV F protein.

16. The fusion protein according to claim 15, wherein the multimerization domain is a foldon domain.

17. The fusion protein according to claim 16 comprising the amino acid sequence of any one of SEQ ID NOs: 10 to 13.

18. A multimer of the mutant RSV F protein according to any one of claims 1 to 14, wherein the fusion protein according to any one of claims 15 to 17 is associated via the multimerization domain.

19. The multimer according to claim 18, wherein the multimer is a trimer.

20. A particulate product of the mutant RSV F protein according to any one of claims 1 to 14 or the multimer according to any one of claims 15 to 17, wherein
the mutant RSV F protein or the fusion protein contains a particle-forming domain,
two or more of the mutant RSV F proteins or the multimers are aggregated via the particle-forming domain to form a particle, and
the particle-forming domain is located at a position closer to an epitope I than to an epitope Φ on a steric structure of the mutant RSV F protein or the multimer.

21. The particulate product according to claim 20, wherein two or more of the mutant RSV F proteins or a fusion proteins for producing a particulate product, in which a particle-forming domain is bound to a C-terminal of the mutant RSV F protein or the fusion protein, are aggregated via the particle-forming domain.

22. The particulate product according to claim 20 or 21, wherein the multimer is a trimer consisting of the fusion protein according to claim 16.

23. The particulate product according to claim 22, wherein the particle-forming domain is an Fc domain consisting of an amino acid sequence of SEQ ID NO: 30, and the particle-forming domain aggregates by binding to a Z domain of a protein A immobilized on a VLP derived from a modified HBs antigen.

24. The particulate product according to claim 22, wherein the particle-forming domain is a peptide consisting of the amino acid sequence of SEQ ID NO: 42.

25. A fusion protein for producing a particulate product comprising: the mutant RSV F protein according to any one of claims 1 to 14, or the fusion protein according to any one of claims 15 to 17, and a particle-forming domain fused to a C-terminal thereof.

26. The fusion protein for producing a particulate product according to claim 25, wherein the particle-forming domain is an Fc domain consisting of the amino acid sequence of SEQ ID NO: 30.

27. The fusion protein for producing a particulate product according to claim 25, wherein the particle-forming domain is a peptide consisting of the amino acid sequence of SEQ ID NO: 42.

28. A polynucleotide encoding the mutant RSV F protein according to any one of claims 1 to 14, the fusion protein according to any one of claims 15 to 17, or the fusion protein for producing a particulate product according to any one of claims 25 to 27.

29. An expression unit comprising the polynucleotide according to claim 28.

30. A host cell comprising the expression unit according to claim 29.

31. An immunogen comprising: the mutant RSV F protein according to any one of claims 1 to 14, the fusion protein according to any one of claims 15 to 17, the multimer according to any one of claims 18 to 19, or the particulate product according to any one of claims 20 to 24.

32. A pharmaceutical composition comprising the expression unit according to claim 29 or the immunogen according to claim 31.

33. An RSV vaccine comprising the expression unit according to claim 29 or the immunogen according to claim 31.

34. The pharmaceutical composition according to claim 32 or the RSV vaccine according to claim 33 which is used for humans.
